# FASCICULE DE BREVET EUROPEEN

(11) **EP 0 428 440 B1**
(45) Date de publication et mention de la délivrance du brevet: **05.03.1997**
(21) Numéro de dépôt: 90403173.9
(22) Date de dépôt: 08.11.1990
(51) Int. Cl.: C07H 17/08, A61K 31/71

(54) **Nouveaux dérivés solubles et non toxiques des macrolides polyéniques basiques, leur préparation et leurs applications**
Lösliche, nichttoxische Derivate von basischen Polyenmakroliden, ihre Herstellung und Verwendung
Soluble, non-toxic derivatives of basic polyene macrolides, their preparation and use

(30) Priorité: 14.11.1989 FR 8914922
(43) Date de publication de la demande: 22.05.1991
(73) Titulaire: MAYOLY-SPINDLER, F-78401 Chatou Cédex (FR); Seman, Michel, F-75011 Paris (FR)
(72) Inventeur: Seman, Michel, F-75011 Paris (FR); Nicolay, Jean-François, F-78400 Chatou (FR)
(74) Mandataire: Ores, Irène

(56) Documents cités:
- US-A- 4 195 172
- POLISH JOURNAL OF CHEMISTRY, vol. 56, 1982, pages 123-130; L. FALKOWSKI et al.: "The structure of N-glycosyl derivatives of polyene macrolide antibiotics. The reaction of nystatin with D-glucose"
- BIOCHEMICAL PHARMACOLOGY, vol. 37, no. 5, 1988, pages 827-836, Pergamon Journals Ltd, GB; M. CHERON et al.: "Quantitative structure-activity relationships in amphotericin B derivatives"

## Description

La présente invention est relative à de nouveaux dérivés solubles et non toxiques des macrolides polyéniques basiques et notamment à de nouveaux amino-1-désoxy-1-cétoses . Elle est également relative au procédé de préparation de ces dérivés et à leur application en tant que médicaments.

Les macrolides polyéniques constituent une classe d'antibiotiques antifongiques présentant un large spectre d'action sur des champignons et des levures pathogènes pour l'homme. Ceci est particulièrement vrai pour l'Amphotéricine B (AmB), antifongique à action fongistatique et fongicide à large spectre : *Candida albicans, Coccidioïdes immitis, Sporotrichum, Cryptococcus neoformans, Histoplasma, Blastomyces, Rhizopus orizae, Aspergillus niger,* etc... Elles sont toujours, malgré l'introduction des imidazoles, le médicament le plus efficace pour bon nombre d'affections.

L'AmB possède en outre des propriétés immunomodulatrices chez la souris et dans une certaine mesure chez l'homme. Elle renforce aussi l'action d'un certain nombre de médicaments anti-cancéraux.

Toutefois, de par sa nature macrocyclique et son caractère amphotère, l'AmB qui répond à la formule A ci-après : 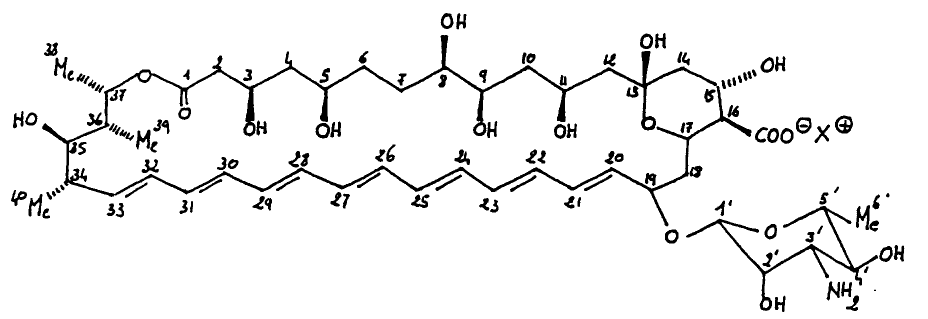 est peu soluble dans l'eau et tend à faire des micelles en solution aqueuse. De plus, la toxicité de l'AmB vis-à-vis des cellules animales et en particulier des cellules rénales, des lymphocytes et des érythrocytes, impose d'importante précautions d'utilisation en clinique. C'est en particulier le cas pour le traitement des mycoses profondes et systémiques qui doivent être traitées par administration intraveineuse.

Ces considérations ont conduit de nombreux chercheurs à rechercher des dérivés non toxiques et plus solubles. On a ainsi réalisé un grand nombre de dérivés, modifiés soit au niveau de la fonction acide en C16, soit au niveau de la fonction amine primaire en C3′. Il y a lieu de citer notamment les travaux de :
- BRUZZESE et al., J. Pharm. sci (1975), 64,462 : estérification du groupe carboxyle ;
- FALKOWSKI et al. Brevet allemand N° 3013631 : amidification du groupe carboxyle
- WRIGHT Brevet Américain N° 4 272 525 : estérification du groupe carboxyle et substitution de l'amine en C3′ par des acides aminés ;
- SCHAFFNER et BOROWSKI, Antib. & Chemo.(1961),II,724 : acétylation de l'amine ;
- SIPOS et KESELSKI Brevet Américain N° 4 235 993 : substitution du groupe amine par un groupe benzyle ;
- KULBAKH et al. Brevet Américain N° 4 007 166 : formation d'un complexe N-méthyl-D-glucamine-macrolide
- FALKOWSKI et al. Brevet Américain N° 4 195 172, Brevet Britannique N° 1 387 187 : glycolisation du groupe amine.

On a également tenté d'augmenter la solubilité de l'Amphotéricine B par addition de tensio-actifs ou par la formation de sels (Brevet Européen 31 722). Malheureusement toutes ces tentatives ont abouti à des dérivés instables en solution ou à des dérivés ayant perdu leurs propriétés antibiotiques.

La présente invention s'est par conséquent donné pour but de pourvoir à de nouveaux dérivés de macrolides polyéniques basiques et notamment de l'AmB qui répondent mieux aux nécessités de la pratique que les dérivés antérieurement connus, en ce qu'ils sont solubles, peu ou pas toxiques, en ce qu'ils conservent le caractère amphotère de l'AmB et en ce qu'ils sont stables en milieu aqueux. L'on sait depuis fort longtemps (Amadori, 1925) qu'en milieu acide, les glycosylamines se transforment en amino-1-désoxy-1-cétoses. Les mêmes composés peuvent être obtenus directement à partir d'un ose et d'une amine en présence d'un catalyseur approprié, suivant le schéma B : 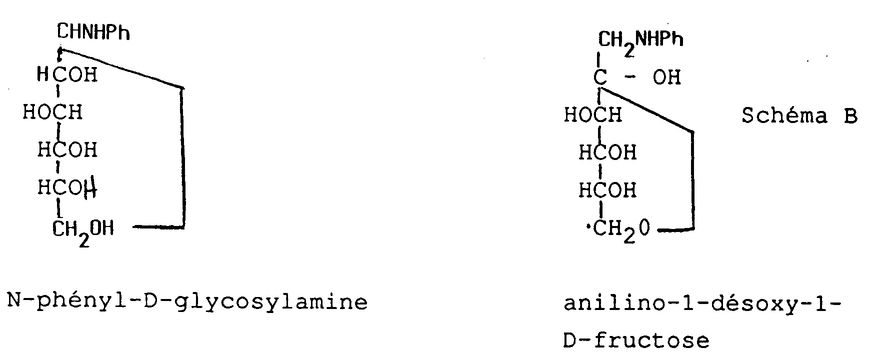 Cet arrangement est dit d'Amadori et a été décrit plus particulièrement dans Adv. Carbohydr. Chem. (1955), 10, 169. Dans leur recherche de dérivés originaux solubles et peu toxiques, qui conservent leur stabilité en solution aqueuse, les Inventeurs ont constaté que, lors de la condensation d'un sucre sur la fonction amine primaire de la mycosamine du macrolide, (qui a été tentée et réalisée par différents chercheurs), la nature du sucre condensé jouait un rôle essentiel sur la solubilité et la stabilité des dérivés obtenus. Si les différents sucres utilisés sont théoriquement tous capables de former une glycosylamine avec l'AmB, il est apparu que les propriétés de solubilité, la stabilité et les propriétés biologiques dépendaient de l'aptitude de la glycosylamine ainsi formée à effectuer un réarrangement d'Amadori.

En ce qui concerne les macrolides polyéniques basiques, un réarrangement amadorien spontané a été observé au cours de la condensation de D-glucose sur la mycosamine du macrolide [FALKOWSKI et al., Polish journal of Chemistry, 56, 123-130, (1982)). Toutefois, cette publication ne mentionne que ce seul sucre, et ne fait état d'aucune propriété particulière du produit résultant du réarrangement.

La présente Invention a pour objet de nouveaux dérivés de macrolides polyéniques basique comprenant un macrolide polyénique basique N-substitué par un groupe amino-1-désoxy-1-cétose, lui même substitué.

Au sens de la présente Invention, on entend par groupe amino-1-désoxy-cétose tout groupe résultant de la condensation d'un sucre réducteur, sur une fonction amine, et présentant une fonctionnalité béta-amino-cétone caractéristique.

Les dérivés conformes à l'Invention répondent à la formule générale I ci-après : 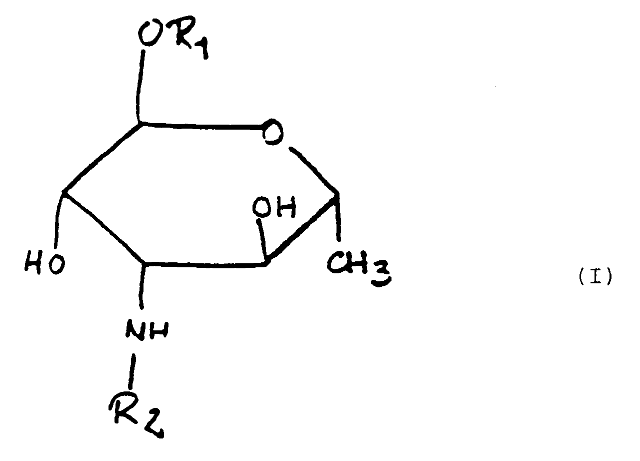 dans laquelle :
R1 désigne la partie macrocyclique d'un macrolide polyénique et
R2 représente :
- une structure répondant à la formule (a) ci-après : 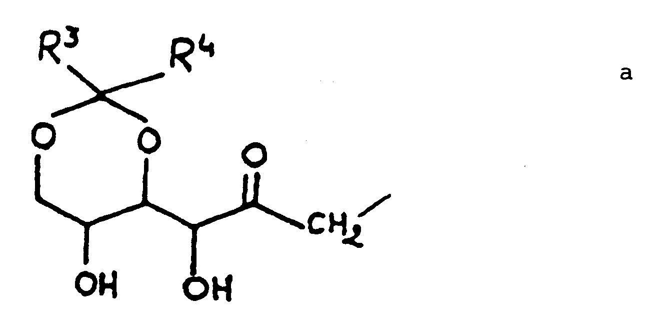 dans laquelle R₃ représente un atome d'hydrogène ou un groupe méthyle,et R₄ représente un atome d'hydrogène, un groupe méthyle, trichlorométhyle, tertiobutyle, propyle, benzyle, phényle, ou phényle substitué par un ou plusieurs groupes méthoxy, nitro ou phosphate, ou bien R₃ et R₄ sont des maillons d'un groupe cycloalkyle, ou bien
- une structure b répondant à la formule ci-après : 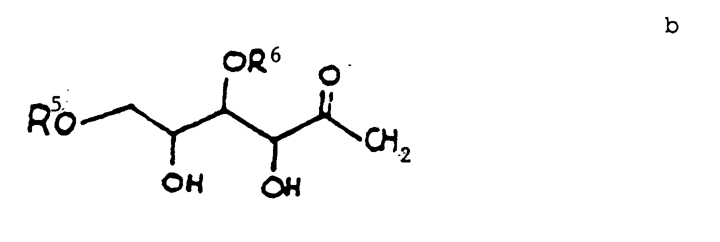 dans laquelle R₅ et R₆ sont des groupes alkyle, aryle, ou acyle, ou bien
- une structure répondant à la formule c ci-après 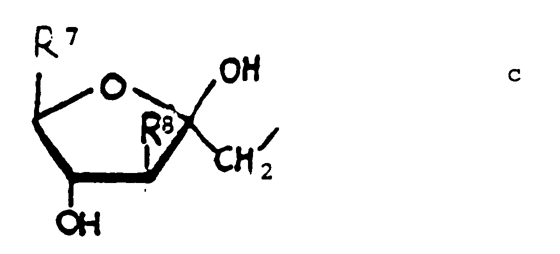 dans laquelle R₇ est un groupe électroattracteur, tel qu'un groupe carboxyle, ester, acyle, amide, nitrile, trihalogénométhyle, ou un groupe hétérocyclique, et R₈ est un groupe hydroxyle, un groupe amine, ou un groupe thiol, ou bien
- une structure de formule d ci-après, dans laquelle R₉, R₁₀, R₁₁, représentent des groupes alkyle, acyle ou aryle, et R₁₂ représente un groupe hydroxyle, amine ou alcoxyle, 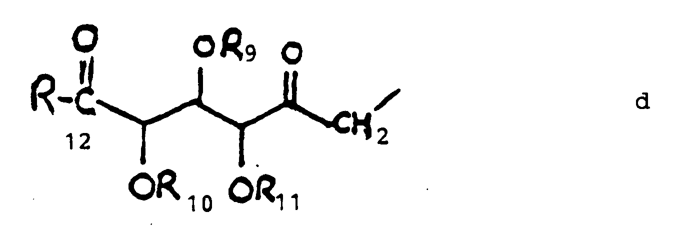
et sont susceptibles d'être obtenus par un procédé comprenant une étape au cours de laquelle on soumet le produit de condensation d'un macrolide polyénique et d'un sucre réducteur, à l'action d'un catalyseur pris dans le groupe constitué par les acides de Brönsted et les catalyseurs à méthylène actif, dans des conditions permettant l'induction d'un réarrangement d'Amadori.

De façon avantageuse, les amino-1-désoxy-1-cétoses conformes à la présente invention, contrairement aux glycosylamines décrites dans l'Art Antérieur précédemment cité qui s'hydrolysent rapidement dans l'eau, sont stables en milieu aqueux neutre ou acide, et présentent
- des propriétés de solubilisation ou de dispersion dans l'eau très satisfaisantes
- les propriétés biologiques de la glycosylamine de départ
- et une cytotoxicité réduite par rapport à celle de la glycosylamine de départ.
Les dérivés conformes à l'invention peuvent être obtenus sous la forme de sels mixtes dont la nature dépend du catalyseur choisi pour le réarrangement, ou de la mise en oeuvre, consécutive audit réarrangement, d'une étape de déplacement, par un autre sel, du sel mixte obtenu.

Suivant un mode de réalisation préféré, les amino-1-désoxy-1-cétoses conformes à l'invention sont sous forme de sels de l'acide de BrÖnsted utilisé pour le réarrangement .

Suivant une modalité particulièrement avantageuse de ce mode de réalisation, les amino-1-désoxy-1-cétoses sont sous forme de sels mixtes d'oxalate et d'ammonium.

Selon un autre mode de réalisation préféré de l'objet de l'invention, les amino-1-désoxy-1-cétoses sont sous forme de sels de N-méthyl glucamine .
La présente invention a également pour objet un procédé de préparation des dérivés de macrolides polyéniques basiques conformes à l'invention, caractérisé en ce qu'au cours d'une première étape on forme une glycosylamine par condensation de la fonction amine du macrolide sur le carbone anomérique d'un sucre réducteur, et en ce qu'au cours d'une deuxième étape on réarrange la glycosylamine obtenue, en milieu acide anhydre, pour former un amino-1-désoxy-1-cétose présentant une fonctionnalité béta amino-cétone caractéristique
et en ce que l'on sépare, au cours d'une troisième étape, l'amino-1-désoxy-1-cétose obtenu du milieu réactionnel.

Suivant un autre mode de réalisation du procédé objet de l'invention, l'étape de condensation a lieu à l'abri de la lumière, sous atmosphère inerte et dans un solvant anhydre, et à une température comprise entre 35°C et 50°C.

Suivant encore un autre mode de réalisation du procédé objet de l'invention, l'étape de réarrangement est induite par l'addition d'un catalyseur choisi dans le groupe qui comprend les acides de Brönsted, tels que, par exemple, l'acide oxalique ou l'acide acétique, et les catalyseurs dits "à méthylène actif", tels que, par exemple, le malonate d'éthyle ou la pentane-2-4-dione.

Selon une modalité préférée de ce mode de réalisation, la séparation du dérivé obtenu est opérée par addition d'une solution aqueuse de sulfate d'ammonium.

Selon une autre modalité préférée de ce mode de réalisation, le sel mixte obtenu peut être déplacé avantageusement par un autre sel. Le sel mixte est suspendu dans le méthanol puis on ajoute volume à volume une solution aqueuse contenant 1 à 3 équivalents d'acide ou de base . L'addition de 3 volumes de butanol permet ensuite d'éliminer l'eau azéotropiquement et de récupérer le produit sous forme cristalline.

La présente invention a également pour objet de nouveaux médicaments, caractérisés en ce qu'ils contiennent en tant que constituant actif, au moins l'un des dérivés de macrolides conformes à la présente invention.

Outre les dispositions qui précèdent, l'invention comprend d'autres dispositions, qui ressortiront du complément de description qui va suivre.

Il doit être bien entendu, toutefois, que ces exemples de préparation, tests et études pharmacologiques sont donnés uniquement à titre d'illustration de l'objet de l'invention, dont ils ne constituent en aucune manière une limitation.

### EXEMPLES DE PREPARATION DE DERIVES DE MACROLIDES DE FORMULE GENERALE I CONFORMES A L'INVENTION

### EXEMPLE 1 : Obtention d'un sel mixte d'oxalate et d'ammonium de 1-amino-1-désoxy-4,6-0-méthyl-benzylidène-D-fructosyl-AmB (AmB40G)

### Formule IV : 1-amino-1-désoxy-4,6-0-méthyl-benzylidène-D-fructosyl-AmB

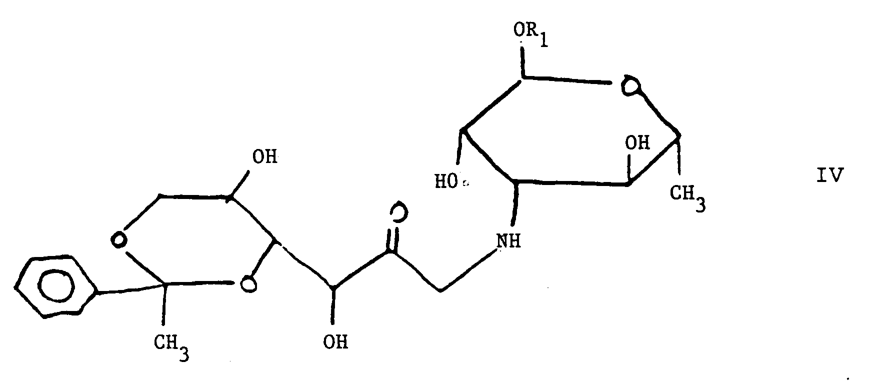

A l'abri de la lumière, sous atmosphère inerte, et en conditions strictement anhydres, l'AmB est mise en suspension dans le diméthyleformamide (anhydre, fraîchement distillé). Sous agitation magnétique, 1,5 équivalents de 4,6-0-méthylbenzylidène-D-glucopyranose sont additionnés, en une fois. On porte la température du mélange à 38°C et on laisse agiter pendant une période pouvant aller de 3 heures jusqu'à 12 heures. Lorsque la solution est parfaitement limpide, le catalyseur est ajouté (un équivalent s'il s'agit d'un acide de Brönsted, tel que l'acide oxalique, 10% du volume pour un "catalyseur à méthylène actif" et on chauffe pendant encore 24h. Le mélange est alors refroidi et le 1-amino-1-désoxy-4,6-0-méthyl-benzylidène-D-fructosyl-AmB est précipité en additionnant sous agitation, au goutte à goutte, une solution à 10% de sulfate d'ammonium dans l'eau. La précipitation est parachevée en laissant la suspension pendant 2H à 4°C. On récupère alors un produit cristallin jaune clair (après centrifugation et élimination du surnageant, que l'on lave 2 fois avec de l'eau (ou un mélange acétone/eau) afin d'éliminer l'excès de carbohydrate de départ et toute trace de solvant.

### Exemple 2 : Obtention de sel de N-méthyl-glucamine de 1-amino-1 désoxy-4,6-0-méthyl-benzylidène-D-fructosyl-AmB (AmB40F)

On prépare le sel de N-méthyl-glucamine de la fonction carboxylique de ce dérivé en suspendant le produit final de l'Exemple 1 dans le méthanol et en ajoutant 2 équivalents d'une base polyhydroxylée (N-méthylglucamine) dissoute dans un volume d'eau équivalent à celui de méthanol. Le mélange devient rapidement limpide et on rajoute alors 3 volumes d'un azéotrope (n-butanol). On évapore sous pression réduite jusqu'à élimination du méthanol et de l'eau. Le dérivé recristallise lentement dans le butanol résiduel et on laisse la suspension à 4°C. Le produit est récupéré après centrifugation, lavé 2 fois avec du butanol puis 2 fois avec de l'éther. Le dérivé est finalement séché sous vide poussé.

### Exemple 3 : Obtention de sel mixte d'oxalate et d'ammonium de 1-amino-1-désoxy-4,6-diméthoxy-fructosyl-AmB (AmB42G)

### Formule V : 1-amino-1-désoxy-4,6-diméthoxy-fructosyl-AmB

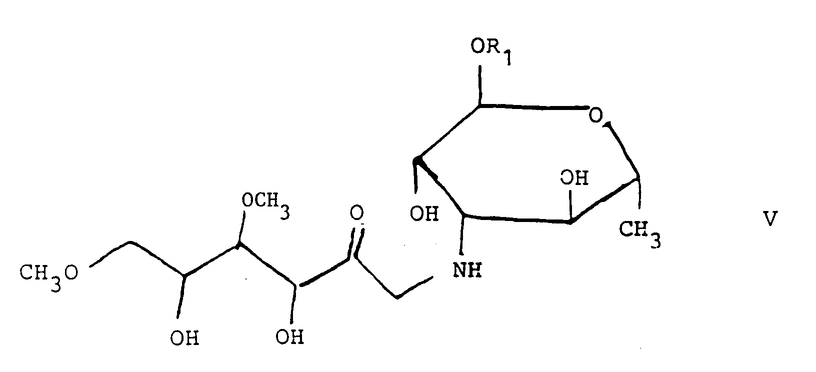

Le procédé pour l'obtention de ce sel est similaire à celui utilisé dans l'Exemple 1 sauf que le carbohydrate couplé au macrolide est le 4,6-di-O-méthyl-D-glucopyranose.

### Exemple 4 : Obtention de sel de N-méthyl glucamine de 1-amino-1-désoxy-4,6-diméthoxy-fructosyl-AmB (AmB42 F)

On prépare le sel de N-méthyl-glucamine de la fonction acide de ce dérivé en suspendant le produit final de l'exemple 3 dans le méthanol puis en suivant le procédé décrit dans l'exemple 2.

### Exemple 5 : Obtention de sel mixte d'oxalate et d'ammonium de 1-désoxy-1-amino-4,6-0-benzylidène-D-fructosyl-AmB (AmB20G)

### Formule VI : 1-désoxy-1-amino-4,6-0-benzylidène-D-fructosyl-AmB

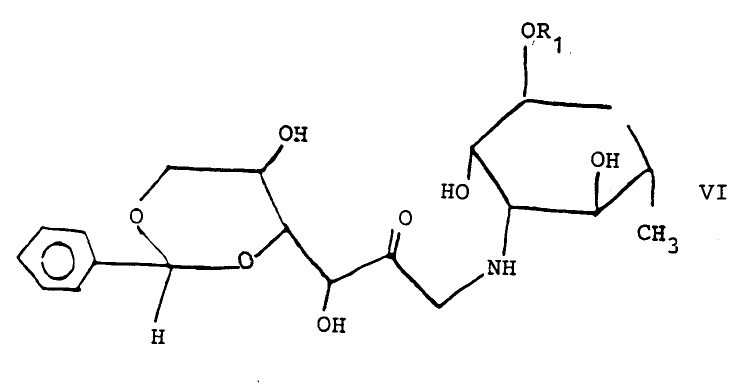

Le procédé utilisé pour l'obtention de ce sel est similaire à celui utilisé dans l'Exemple 1 sauf que le carbohydrate couplé au macrolide est le 4,6-0-benzylidène-D-glucopyranose.

### Exemple 6 : Obtention de sels de N-méthyl-glucamine de 1 désoxy-1 amino-4,6-0-benzylidène-D-fructosyl-AmB (AmB20F)

On prépare le sel de N-méthyl-glucamine de la fonction acide de ce dérivé en suspendant le produit final de l'exemple 5 dans le méthanol puis en suivant le procédé décrit dans l'exemple 2.

### Exemple 7 : Obtention de sels mixtes d'oxalate et d'ammonium de 1-désoxy-1-amino-4,6-O-éthylidène-D-fructosyl-AmB (AmB22G)

### Formule VII : 1-désoxy-1-amino-4,6-O-éthylidène-D-fructosyl-AmB

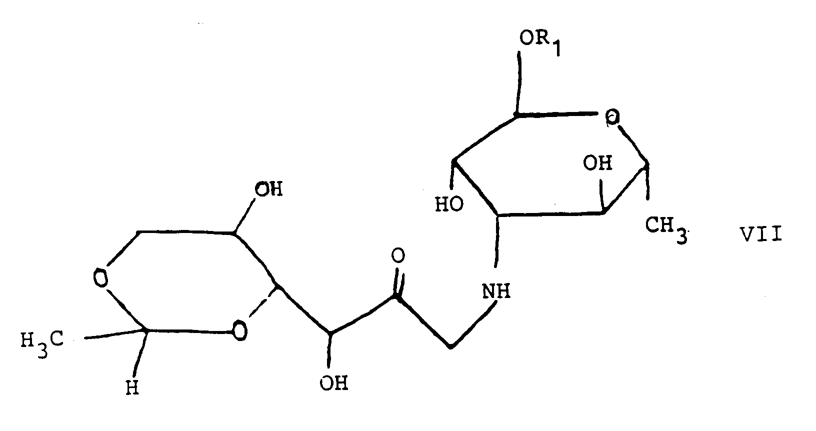

Le procédé utilisé pour l'obtention de ce sel est similaire à celui utilisé dans l'exemple 1 sauf que le carbohydrate couplé au macrolide est le 4,6-0-éthylidène-D-glucopyranose.

### Exemple 8 : Obtention de sel de N-méthyl-glucamine de 1-désoxy-1 amino- 4,6-0-éthylidène-D-fructosyl-AmB (AmB22F)

On prépare le sel de N-méthyl-glucamine de la fonction acide de ce dérivé en suspendant le produit final de l'exemple 7 dans le méthanol puis en suivant le procédé décrit dans l'exemple 2.

### Exemple 9 : Obtention de sels mixtes d'oxalate et d'ammonium de 6-désoxy-6-N-acétyl-1-désoxy-1-aminofructosyl-AmB (AmB23G)

### Formule VIII : 6-désoxy-6-N-acétyl-1-désoxy-1-aminofructosyl-AmB

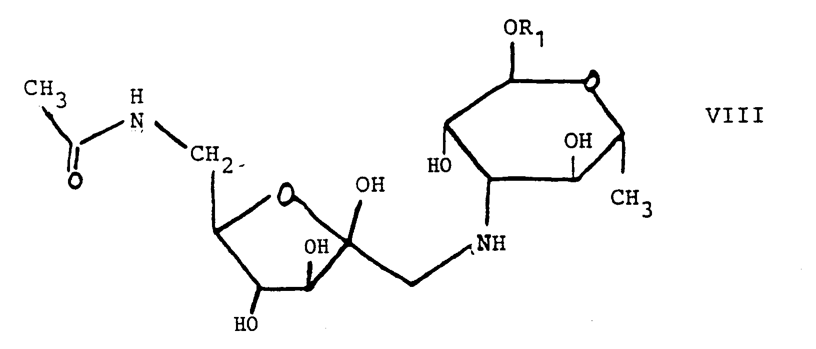

Le procédé utilisé pour l'obtention de ce sel est similaire à celui utilisé dans l'Exemple 1 sauf que le carbohydrate couplé au macrolide est le 6-désoxy-6-N-acétyl-D-glucopyranose.

### Exemple 10 : Obtention de sels de N-méthyl-glucamine de 6 désoxy-6 N-acétyl-1 désoxy-1 amino-fructosyl AmB (AmB23F)

On prépare le sel de N-méthyl-glucamine de la fonction acide de ce dérivé en suspendant le produit final de l'exemple 9 dans le méthanol puis en suivant le procédé décrit dans l'exemple 2.

### Exemple 11 : Obtention de sels mixtes d'oxalate et d'ammonium de 1-amino-désoxy-D-arabino-glucuronyl-AmB par condensation de D-glucurono-6,3-lactone et d'AmB. (AmB 17 G)

### Formule IX : 1-amino-désoxy-D-arabino-glucuronyl-AmB

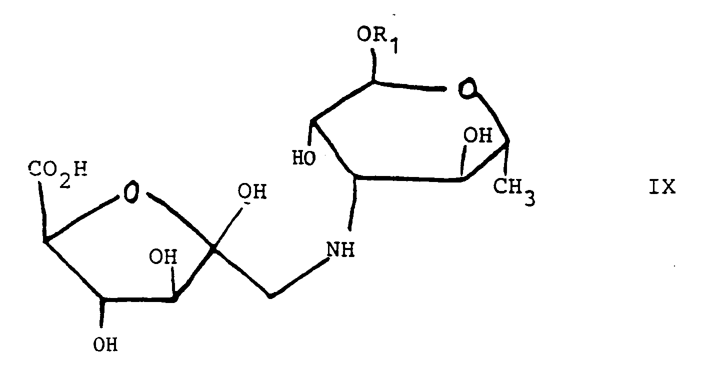

Le procédé utilisé pour l'obtention de ce sel est similaire à celui utilisé dans l'Exemple 1 sauf que le carbohydrate couplé au macrolide est le D-glucurono-6,3-lactone.

### Exemple 12 : Obtention de sels de N-méthyl-glucamine de 1-amino-désoxy-D arabino-glucuronyl-AmB par condensation de D-glucurono-6,3-lactone et d'AmB. (AmB 17 F)

On prépare le sel de N-méthyl-glucamine de la fonction acide de ce dérivé en suspendant le produit final de l'exemple 11 dans le méthanol puis en suivant le procédé décrit dans l'exemple 2.

### Exemple 13 : Obtention de sels mixtes d'oxalate et d'ammonium de 1-amino-1-désoxy-D-arabino-glucuronamidyl-AmB (AmB 12 G)

### Formule X : 1-amino-1-désoxy-D-arabino-glucuronamidyl-AmB

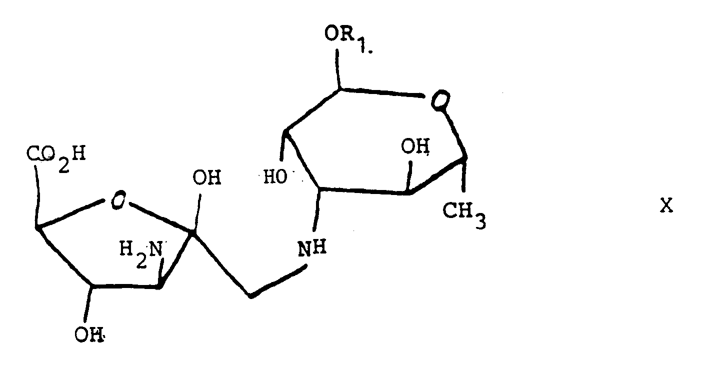

Le procédé utilisé pour l'obtention de ce sel est similaire à celui utilisé dans l'Exemple 1 sauf que le carbohydrate couplé au macrolide est le glucuronamide.

### Exemple 14 : Obtention de sels de N-méthyl-glucamine de 1-amino-1-désoxy-D-arabino-glucuronamidyl-AmB (AmB 12 F)

On prépare le sel de N-méthyl-glucamine de la fonction acide de ce dérivé en suspendant le produit final de l'exemple 13 dans le méthanol puis en suivant le procédé décrit dans l'exemple 2.

### Exemple 15 : Obtention de sels mixtes d'oxalate et d'ammonium de 1,3-di-amino-1,3-di-désoxy-D-arabino-glucuronamidyl-AmB (AmB50G).

### Formule XI : 1,3-di-amino-1,3-di-désoxy-D-arabino-glucuronyl-AmB

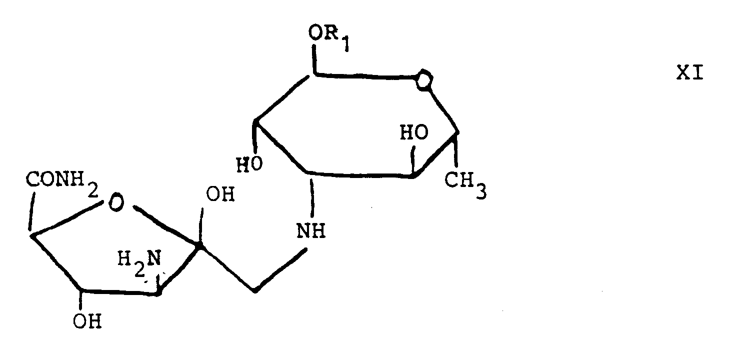

Le procédé utilisé pour l'obtention de ce sel est similaire à celui utilisé dans l'Exemple 1 sauf que le carbohydrate couplé au macrolide est le 3-amino-3-désoxy-D-glucurono-6,3-lactame.

### Exemple 16 : Obtention de sels de N-méthyl-glucamine de 1,3-di-amino-1,3-di-désoxy-D-arabino-glucuronyl-AmB (AmB50F)

On prépare le sel de N-méthyl-glucamine de la fonction acide de ce dérivé en suspendant le produit final de l'exemple 15 dans le méthanol puis en suivant le procédé décrit dans l'exemple 2.

### Exemple 17 : Obtention de sels mixtes d'oxalate et d'ammonium de 1-amino-1-désoxy-D-arabino-glucuronyl-AmB (AmB17G) par condensation d'acide glucuronique et d'AmB

Le procédé utilisé pour l'obtention de ce sel est similaire à celui utilisé dans l'Exemple 1 sauf que le carbohydrate couplé au macrolide est l'acide D-glucuronique. Le produit obtenu est identique à celui de l'exemple 11.

### COMPTE RENDU D'ETUDES MICROBIOLOGIQUES ET TOXICOLOGIQUES REALISES POUR METTRE EN EVIDENCE LES PROPRIETES THERAPEUTIQUES DES DERIVES DE L'AmB DE FORMULE GENERALE I

### A) ETUDES MICROBIOLOGIQUES

### A.1 : Etude de l'efficacité antibiotique en milieu liquide de dérivés de l'AmB sur différentes levures

L'efficacité antibiotique a été testée selon la méthode d'inhibition de croissance en milieu liquide (bouillon Sabouraud) en présence de différentes concentrations des dérivés étudiés.

Une suspension de levures a été mise en croissance dans du bouillon de façon à obtenir environ 1.10² cellules/ml.

Les tubes contenant les différentes concentrations de substance antifongique ont été ensemencés avec une même quantité de cellules de levures puis ils ont été incubés à 28°C avec agitation pendant 18 heures. Pour chaque concentration de substance antifongique deux tubes identiques ont été préparés.

L'inhibition de croissance fongique selon les différentes concentrations a été évaluée pour chaque molécule en spectrophotométrie à 620 nm et exprimée en pourcentage par rapport à un témoin sans antifongique.

### A.2 : Etude de l'efficacité antibiotique en milieu gélosé de dérivés de l'Amphotéricine B sur Aspergillus

L'activité antibiotique a été testée selon la technique de diffusion en milieu gélosé (gélose Sabouraud).

L'efficacité de chaque molécule a été déterminée par la taille de la zone d'inhibition de croissance autour d'un disque de papier WHATMAN 3M imbibé par la substance antibiotique à une concentration donnée.

Une suspension de spores d'*Aspergillus* a été préparée en bouillon, 0,2 ml de cette suspension ont été ensemencé en surface sur le milieu gélosé solidifié de façon à obtenir une nappe homogène de croissance. Une fois l'étalement effectué les disques de papier WHATMAN 3M (diamètre : 1 cm) ont été déposés sur la surface de la gélose puis ont été imbibés de 0,1 ml de substance antibiotique à différentes concentrations.

Les doses donnant 50% d'inhibition de la croissance des espèces *S. cerevisiae, C. neoformans, P. orbiculare, C. albicans, C. albicans B 2630*, et les doses donnant une zone d'inhibition de la croissance des espèces *A. flavus et A. fumigatus*, de 1 cm autour d'un disque déposé sur milieu de culture solide, déterminées dans les deux cas avec les dérivés 12, 17, 20, 22 et 23 des séries G et F, sont représentées sur les Tableaux 1 et 1bis ci-après. Ces tableaux montrent que les dérivés de l'AmB préparés selon l'invention conservent donc les propriétés antibiotiques de la molécule dont ils sont issus, l'AmB.

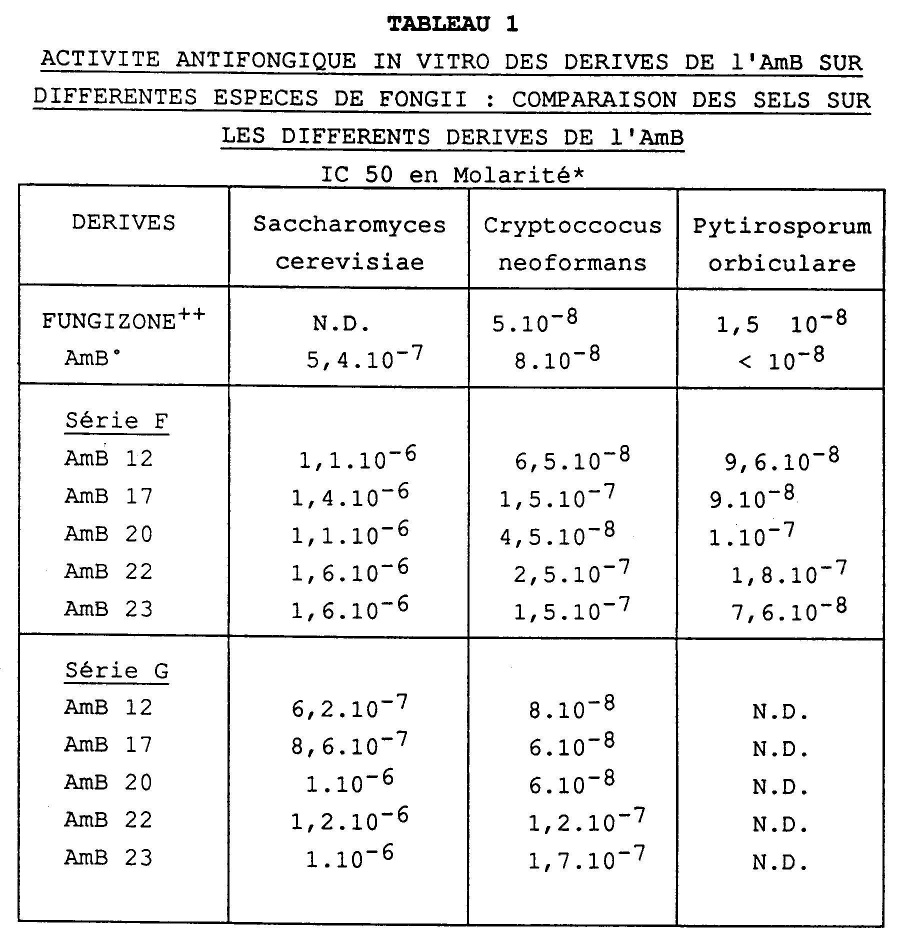

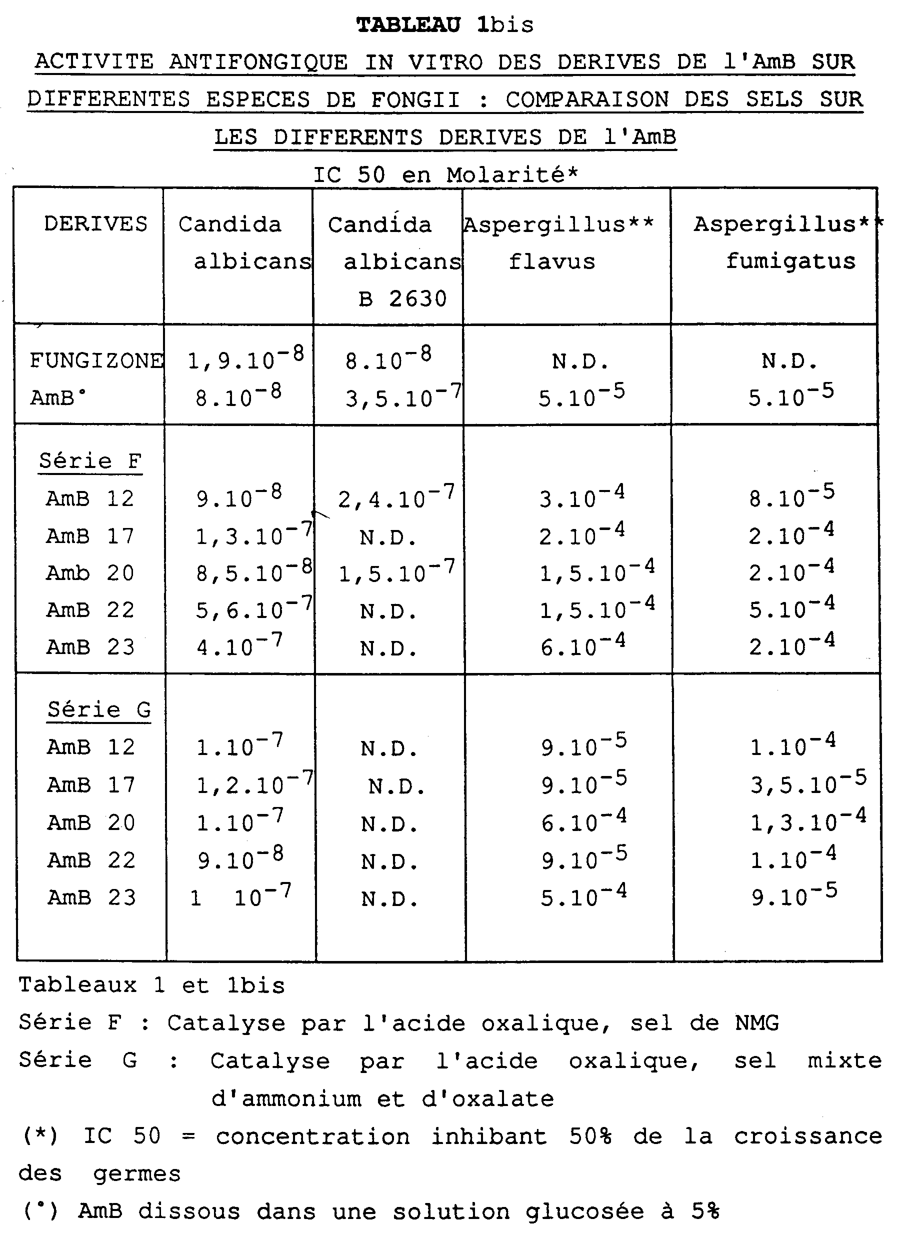

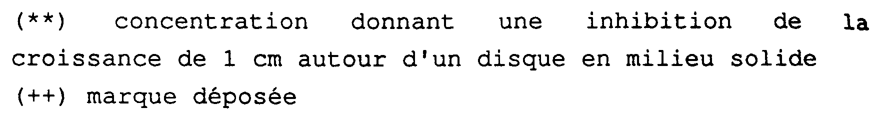

### B) ETUDES TOXICOLOGIQUES

Les propriétés toxiques des dérivés ont été étudiées sur des globules rouges humains et murins ainsi que sur des cellules lymphoïdes du sang périphérique, de la moelle osseuse, du Thymus chez l'Homme, sur les lymphocytes T et B sphériques de la souris et sur deux lignées tumorales : XG3 (souris), Daudi (Homme).

### B.1 : Etude de la toxicité sur les globules rouges

Toutes les solutions des dérivés de l'AmB conformes à l'invention sont préparées extemporanément en diluant 4mg des molécules dans 100 µl de solution de glucose à 5% puis, après 15 minutes à l'abri de la lumière, on ajoute 100 µl d'eau distillée stérile et on complète avec une solution de glucose à 5% pour obtenir des solutions mères à 4mg/ml. Pour étudier comparativement les effets de l'AmB, 4 mg de FUNGIZONE sont dilués dans 1 ml de glucose à 5%.

Les cellules sont préparées en déposant 1ml de sang périphérique prélevé et dilué au demi sur du Ficollhypaque pour éliminer les lymphocytes, puis en centrifugeant 20 min. à 2000 rpm. Le culot de globules rouges est récupéré, lavé une fois en NaCl à 0,9% puis deux fois en KCl 150mM, Tris HCl 0,5mM, pH 7,4.

On fait ensuite une suspension à 1,25% dans une solution KCl 150mm, Tris HCl 0,5mm, pH 7,4 et on en distribue 150 µl par puits ainsi que 50 µl de chacune des dilutions de dérivés, plus un puits de contrôle (solution de glucose à 5%).

Les cellules sont alors incubées 1 heure 30 à 37°C en étuve humide.

Pour évaluer la lyse cellulaire, l'hémoglobine est dosée en prélevant 100 µl de surnageant dans chaque puits et en les diluant dans 1ml d'eau distillée.

On calcule la concentration en hémoglobine en mesurant la densité optique à 540nm et en tenant compte de l'absorption des dérivés de l'Amphotéricine B.

La DL 50 est la dose qui entraîne la lyse de 50% des hématies (un témoin de lyse de 100% est obtenu avec l'eau distillée, un témoin de lyse de 0% est obtenu avec la solution de glucose à 5%).

### B.2 : Etude de la toxicité sur les cellules nucléées

Les solutions de dérivés de l'Amphotéricine B sont préparées d'une façon similaire à celles mentionnées au paragraphe B.1.

Les cellules sont préparées en déposant 20 ml de sang dilué au 1/2 avec du NaCl 0,9%, sur 10 ml de Ficoll-hypaque et en centrifugeant 30mn à 2000 pm. L'anneau de cellules est récupéré et lavé deux fois en tampon PBS contenant 5% de sérum de veau foetal.

Les cellules sont préparées en suspendant 1,14.10⁶ cellules/ml dans du RPMI 1640 contenant 2,5% de sérum de veau foetal et en distribuant 150 µl par puits. 50 µl de chaque dilution des dérivés sont ajoutés ainsi qu'un contrôle (solution de glucose à 5%).

Les cellules sont ensuite incubées 1h30 dans une étuve humide à 5% de CO₂, à 37°C ou à une température ambiante.

Afin d'évaluer la toxicité des dérivés, on fait une numération cellulaire en présence de bleu de tryptan et on calcule la DL 50 (concentration de dérivé entraînant 50% de mortalité) par rapport au contrôle (solution de glucose à 5%) pour chaque concentration de chaque dérivé.

Les tableaux 2, 2bis et 3, 3bis ci-après montrent que les dérivés préparés selon l'Invention sont beaucoup moins toxiques que l'Amphotéricine B pour les cellules humaines et murines, ceci pouvant varier d'un dérivé à l'autre. Le tableau 4 représente une étude comparative in vitro de la toxicité de l'AmB, du dérivé non réarrangé, et du même dérivé réarrangé en utilisant différents catalyseurs.

En conclusion, ces études montrent que les dérivés obtenus selon la présente invention conservent les propriétés antibiotiques de l'AmB et que leur toxicité a été perdue totalement ou a diminué considérablement.

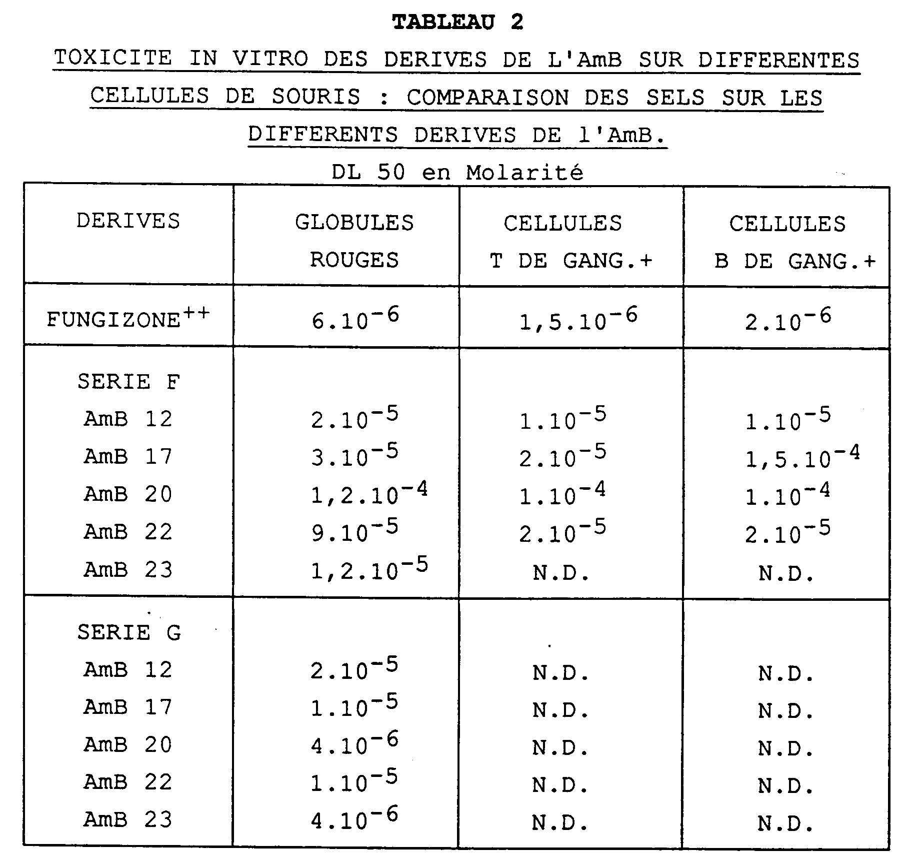

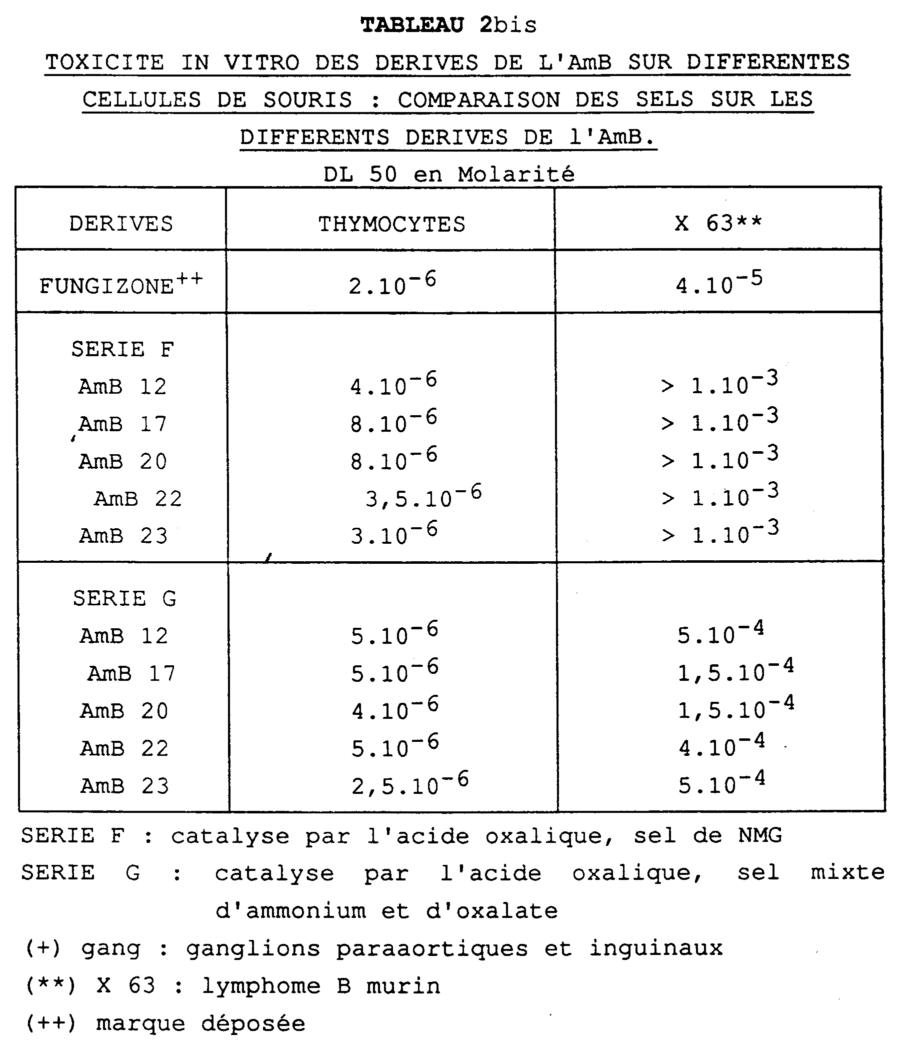

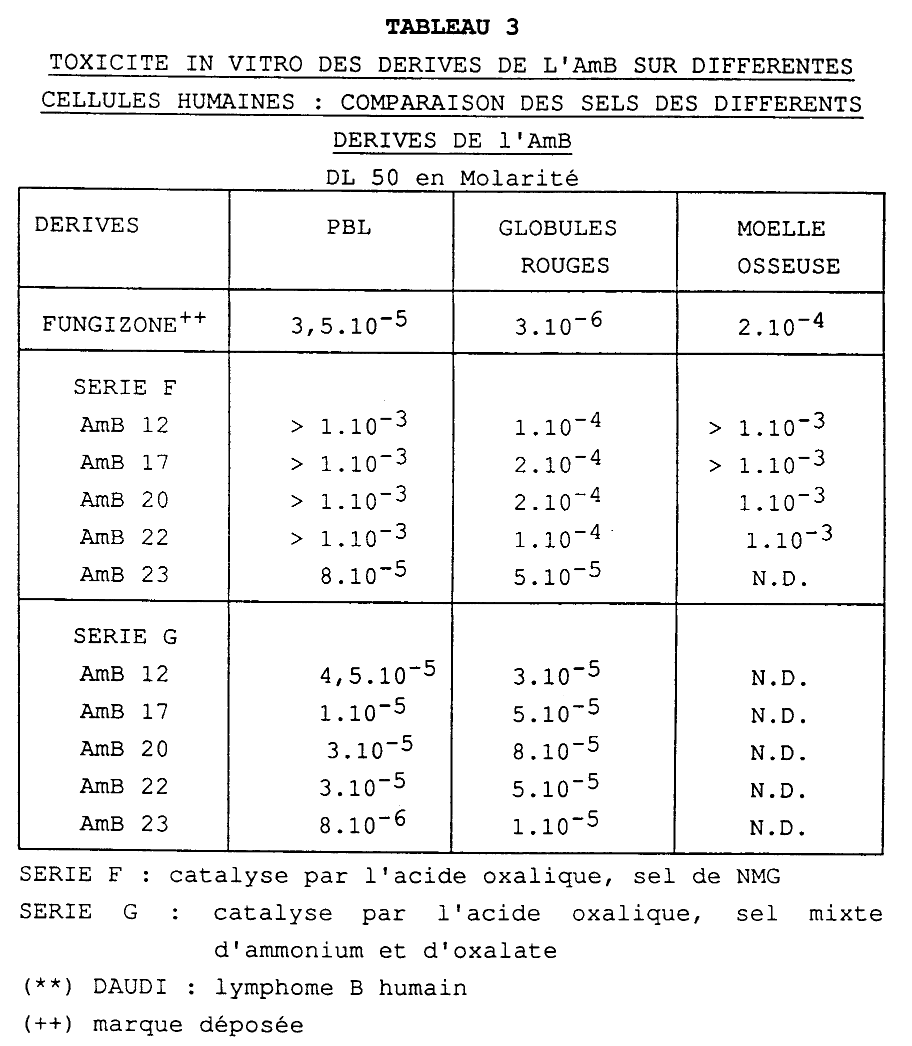

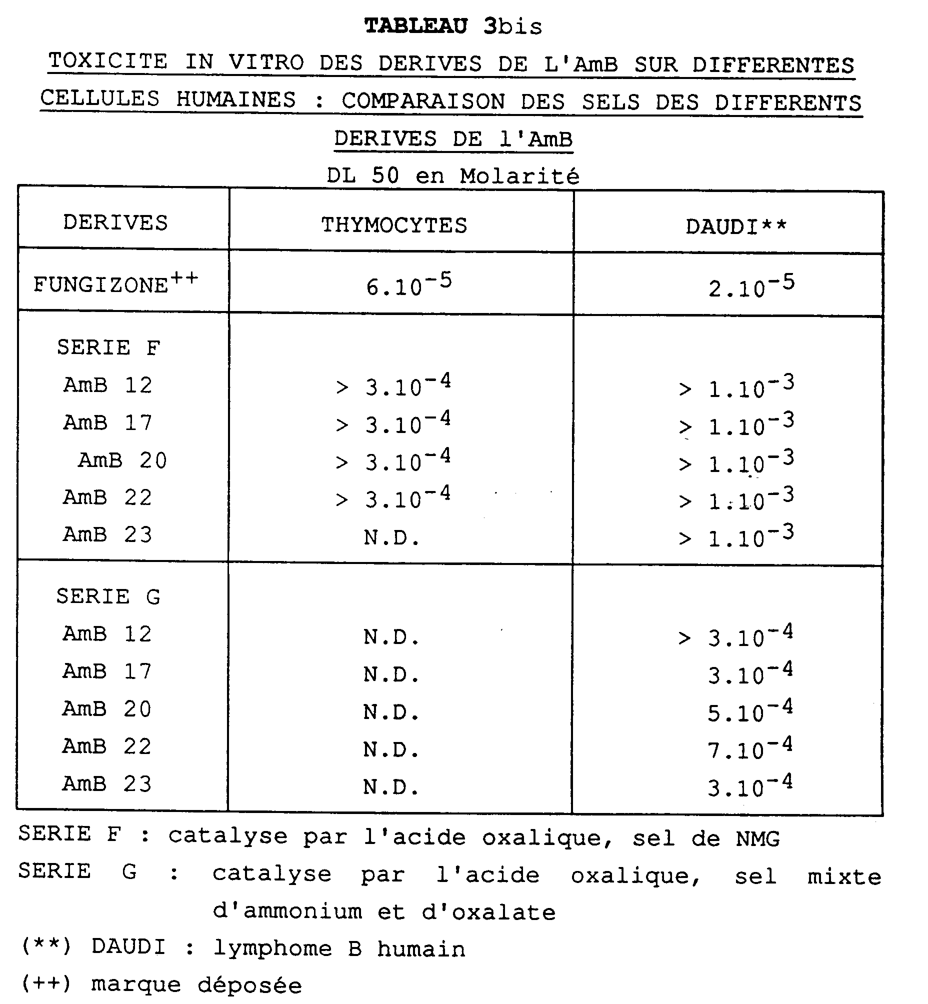

**TABLEAU 4**

| TOXICITE IN VITRO DU DERIVE AmB 12 SUR LES PBLs HUMAINES: COMPARAISON DE DIFFERENTS CATALYSEURS | |
|---|---|
| DERIVE | DL 50 |
| FUNGIZONE⁺⁺ | 3,5.10⁻⁵ |
| Sans catalyse^{(a)} sel de NMG | 8.10⁻⁵ |
| Malonate d'éthyle^{(b)} sel de NMG | > 1.10⁻³ |
| acide oxalique^{(b)} sel de NMG | > 1.10⁻³ |
| acide acétique^{(b)} sel de NMG | 2.10⁻⁴ |

| | |
|---|---|
| (a) dérivé non réarrangé | |
| (b) catalyseur utilisé pour le réarrangement | |
| (++) marque déposée | |

### C) ETUDE DE LA STRUCTURE ET DE LA STABILITE DES DERIVES SELON LA PRESENTE INVENTION

### C.1 Etude analytique de la structure

### C.1.a Spectre UV

Les spectres UV des dérivés d'AmB selon l'invention montrent la conservation de la structure polyénique de l'AmB.

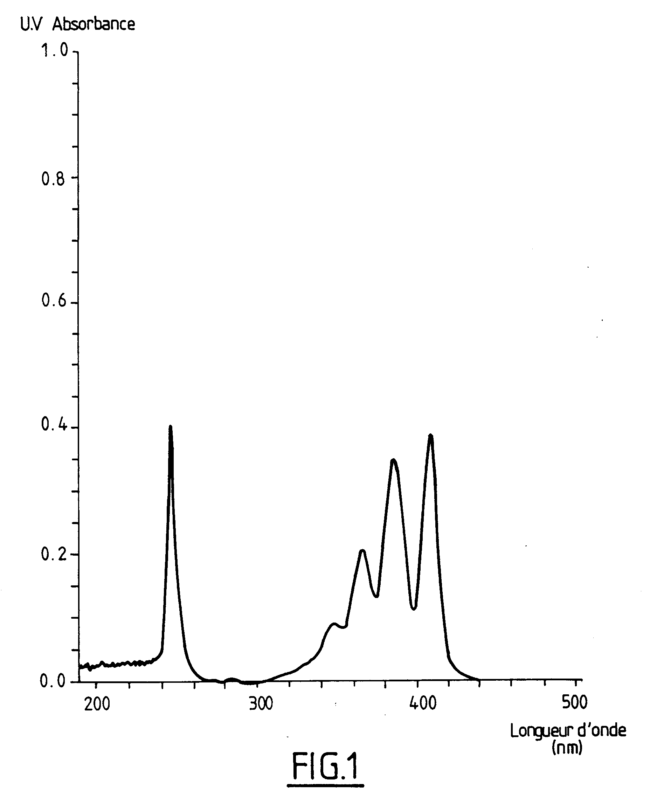

La figure 1 représente en exemple le spectre UV de l'AmB 12 F.

### C.1.b RMN du carbone

Les spectres RMN ¹³C des dérivés selon l'invention permettent de confirmer leur structure amadorienne.

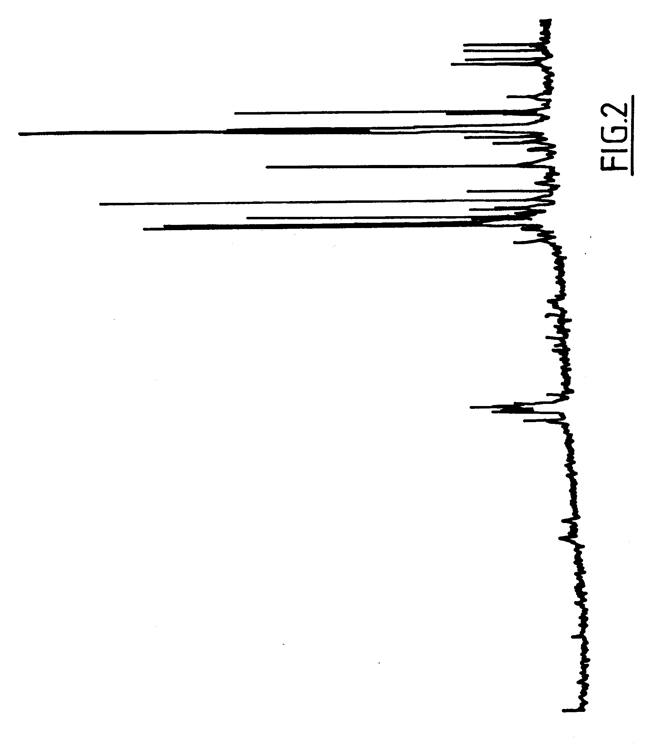

Le spectre ¹³C RMN de l'AmB 12 F est donné en exemple, figure 2.

### C.1.c: HPLC

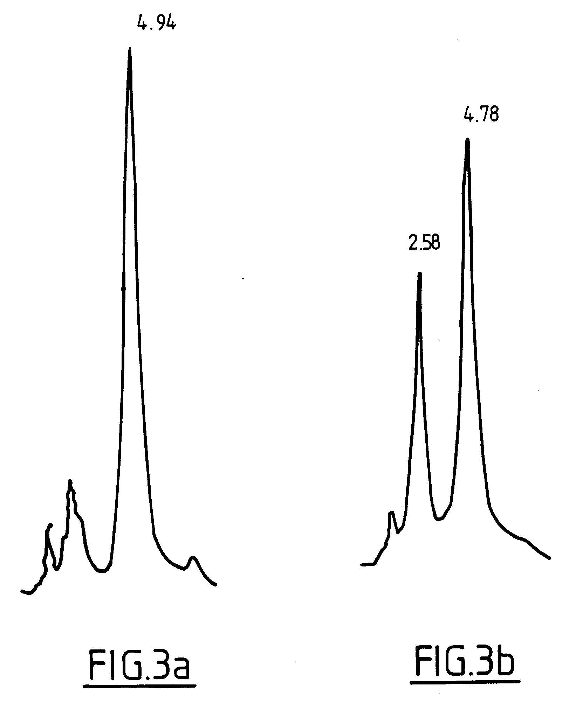

Les spectres HPLC en phase inverse du dérivé AmB 12 réarrangé et du dérivé correspondant non réarrangé sont donnés en exemple à la figure 3.

### C.2 Solubilité des dérivés d'AmB suivant l'invention

L'AmB (utilisé en tant que témoin) ou les dérivés obtenus sont dissous à température ambiante dans une solution de glucose à 5%, jusqu'à saturation.

Dans ces conditions, l'AmB est insoluble.

Le dérivé AmB 17 est soluble jusqu'à une concentration de 20 mg/ml.

Le dérivé AmB 20 est soluble jusqu'à une concentration de 40 mg/ml.

Le dérivé AmB 12 est soluble jusqu'à une concentration de 150 mg/ml.

### C.3 Stabilité des dérivés d'AmB suivant la présente invention

### C.3.a. : en solution

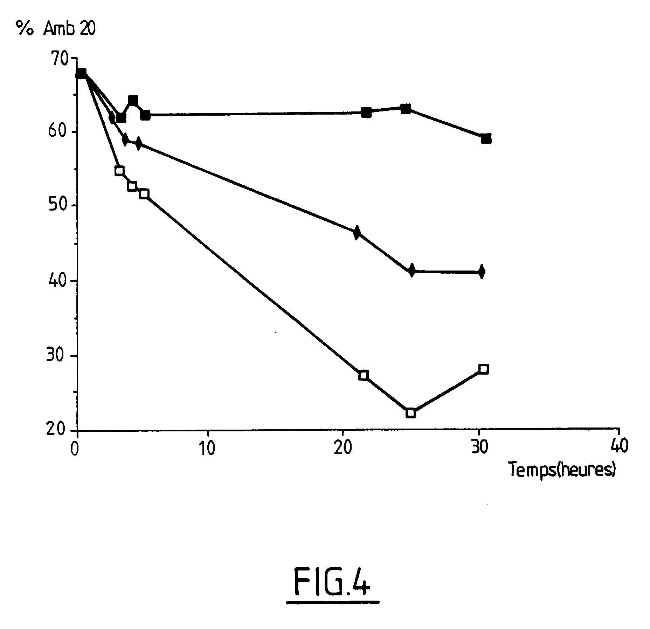

La dégradation des dérivés au cours du temps est suivie par HPLC. La figure 4 montre les résultats obtenus (pourcentage du dérivé non dégradé) pour une solution à 2,5 mg/ml d'AmB 20 à :
- □: 37°C
- ◆: température ambiante
- ■: 4°C

### C.3.b. : sous forme de poudre

Lorsque les dérivés selon la présente invention sont conservés sous forme de poudre, ils restent stables pendant plusieurs mois.

## Revendications (Revendications pour l'(les) Etat(s) contractant(s) suivant(s): DE, GB, FR, IT, NL, SE, LI, CH, BE, AT, LU, DK)

1. Dérivés de macrolides polyéniques basiques, caractérisés en ce qu'ils répondent à la formule générale (I) ci-après : 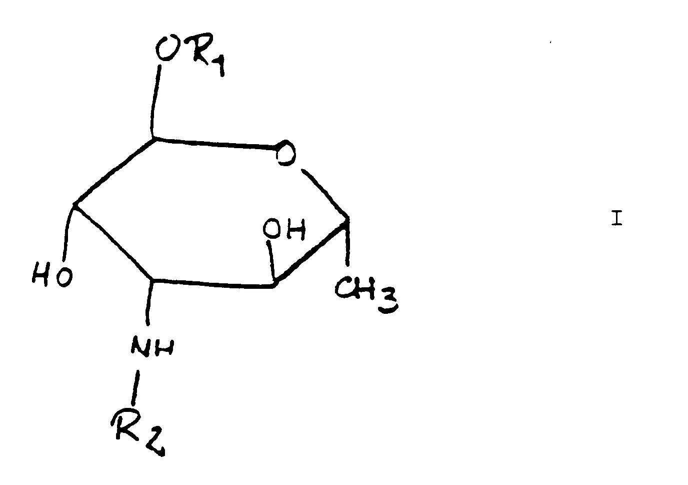 dans laquelle :
R₁ désigne la partie macrocyclique d'un macrolide polyénique et
R₂ représente :
- une structure répondant à la formule (a) ci-après : 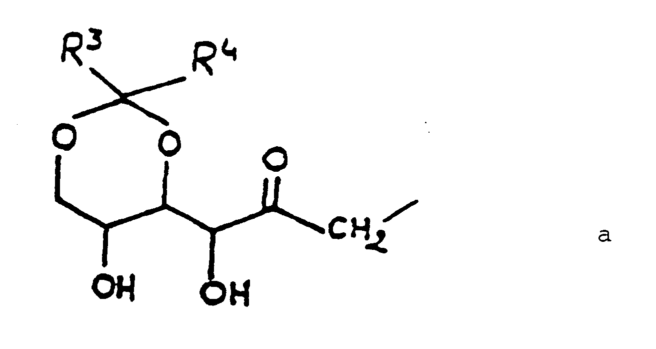 dans laquelle R₃ représente un atome d'hydrogène ou un groupe méthyle, et R₄ représente un atome d'hydrogène, un groupe méthyle, trichlorométhyle, tertiobutyle, propyle, benzyle, phényle, ou phényle substitué par un ou plusieurs groupes méthoxy, nitro ou phosphate, ou bien R₃ et R₄ sont des maillons d'un groupe cycloalkyle, ou bien
- une structure répondant à la formule (b) ci-après : 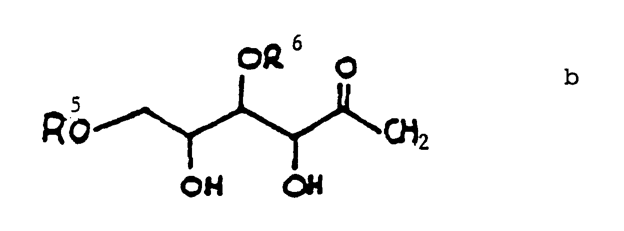 dans laquelle R₅ et R₆ sont des groupes alkyle, aryle, ou acyle, ou bien
- une structure répondant à la formule (c) ci-après : 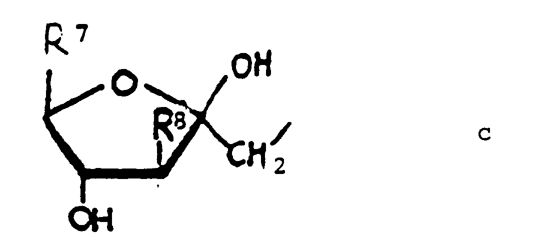 dans laquelle R₇ est un groupe carboxyle, ester, acyle, amide, nitrile, trihalogénométhyle, ou un groupe hétérocyclique, et R₈ est un groupe hydroxyle, un groupe amine, ou un groupe thiol ;
ou bien
- une structure de formule (d) ci-après, dans laquelle R₉, R₁₀, R₁₁, représentent des groupes alkyle, acyle ou aryle, et R₁₂ représente un groupe hydroxyle, amine ou alcoxyle, 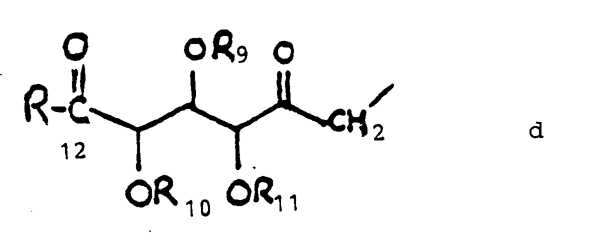
et en ce qu'il est susceptible d'être obtenu par un procédé comprenant une étape au cours de laquelle on soumet le produit de condensation d'un macrolide polyénique et d'un sucre réducteur, à l'action d'un catalyseur pris dans le groupe constitué par les acides de Brönsted et les catalyseurs à méthylène actif, dans des conditions permettant l'induction d'un réarrangement d'Amadori.

2. Dérivé selon la revendication 1, caractérisé en ce que ledit dérivé est sous forme de sel d'un acide de Brönsted.

3. Dérivé selon la revendication 2, caractérisé en ce que ledit dérivé est sous forme de sel mixte d'oxalate et d'ammonium.

4. Dérivé selon la revendication 1, caractérisé en ce que ledit dérivé est sous forme de sel de N-méthyl glucamine.

5. Dérivé selon l'une quelconque des revendications 1 à 4, caractérisé en ce que le macrolide polyénique est l'Amphotéricine B.

6. Dérivé selon l'une quelconque des revendications 1 à 5, caractérisé en ce qu'il est le 1-amino-1-désoxy-D-arabino-glucuronyl-AmB répondant à la formule (IX) suivante : 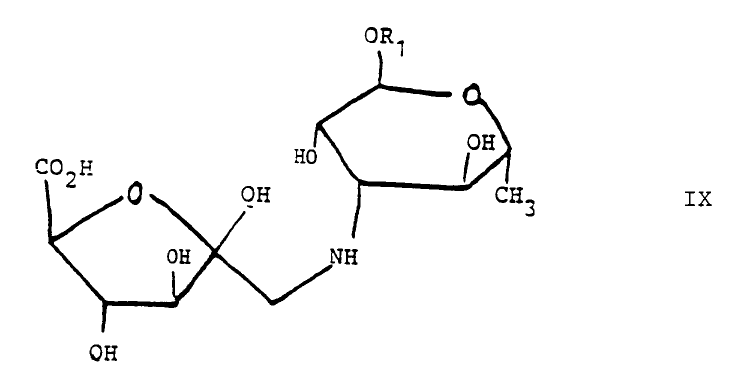 ou son sel de N-méthyl glucamine, ou son sel mixte d'oxalate et d'ammonium.

7. Dérivé selon l'une quelconque des revendications 1 à 5, caractérisé en ce qu'il est le 1-amino-1-désoxy-D-arabino-glucoronamidyl-AMB répondant à la formule (X) ci-après : 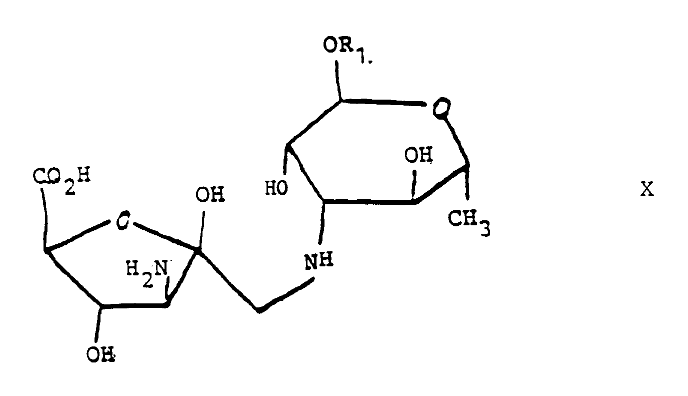 ou son sel de N-méthyl-glucamine, ou son sel mixte d'oxalate et d'ammonium.

8. Dérivé selon l'une quelconque des revendications 1 à 5, caractérisé en ce qu'il est le 1-désoxy-1-amino-4,6-0-benzylidène-D-fructosyl AMB de formule (VI) ci-après : 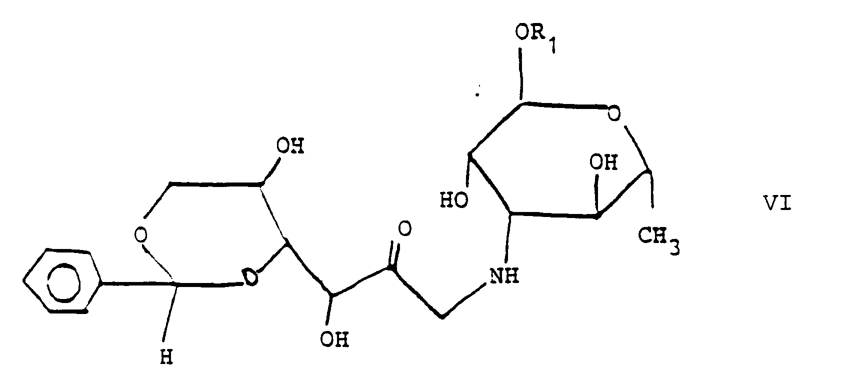 ou son sel de N-méthyl-glucamine, ou son sel mixte d'oxalate et d'ammonium.

9. Procédé de préparation d'un dérivé selon l'une quelconque des revendications 1 à 8, caractérisé en ce qu'il comprend la combinaison des étapes suivantes, dans l'ordre suivant :
a) une étape de condensation d'un macrolide polyénique et d'un sucre réducteur, éventuellement substitué apte à effectuer un rearrangement d'Amadori de façon résultant au résidu R₂ défini dans la revendication 1;
b) une étape d'induction du réarrangement d'Amadori par un catalyseur pris dans le groupe constitué par les acides de Brönsted et les catalyseurs à méthylène actif;
c) une étape de séparation du dérivé réarrangé du milieu réactionnel.

10. Procédé selon la revendication 9, caractérisé en ce qu'il comprend en outre une étape de préparation du sel de N-méthyl-glucamine du dérivé réarrangé.

11. Procédé selon la revendication 9, caractérisé en ce que le sucre réducteur est le D-glucopyranose ou un de ses dérivés.

12. Procédé selon la revendication 9, caractérisé en ce que le sucre réducteur est l'acide β-D-glucuronique ou un de ses dérivés.

13. Médicaments, caractérisés en ce qu'ils contiennent, en tant que principe actif, un dérivé selon l'une quelconque des revendications 1 à 8.

## Revendications (Revendications pour l'(les) Etat(s) contractant(s) suivant(s): ES, GR)

1. Procédé de préparation de dérivés de macrolides polyéniques basiques, caractérisés en ce que l'on prépare des dérivés répondant à la formule générale (I) ci-après : 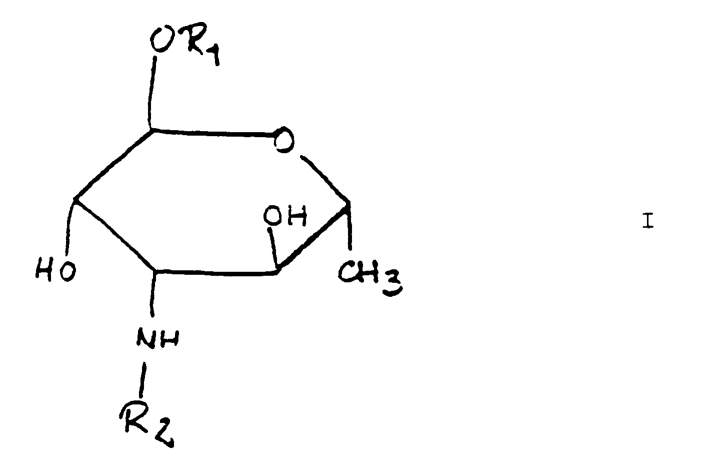 dans laquelle :
R₁ désigne la partie macrocyclique d'un macrolide polyénique, et
R₂ représente :
- une structure répondant à la formule (a) ci-après : 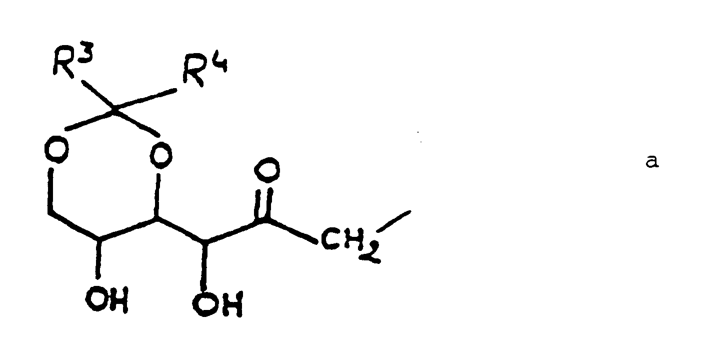 dans laquelle R₃ représente un atome d'hydrogène ou un groupe méthyle, et R₄ représente un atome d'hydrogène, un groupe méthyle, trichlorométhyle, tertiobutyle, propyle, benzyle, phényle, ou phényle substitué par un ou plusieurs groupes méthoxy, nitro ou phosphate, ou bien R₃ et R₄ sont des maillons d'un groupe cycloalkyle,
ou bien
- une structure répondant à la formule (b) ci-après : 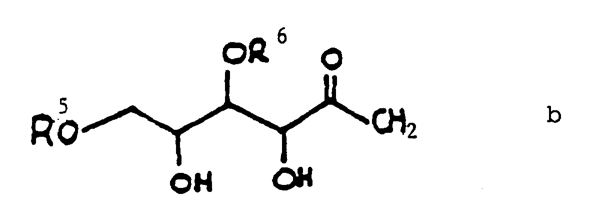 dans laquelle R₅ et R₆ sont des groupes alkyle, aryle, ou acyle, ou bien
- une structure répondant à la formule (c) ci-après : 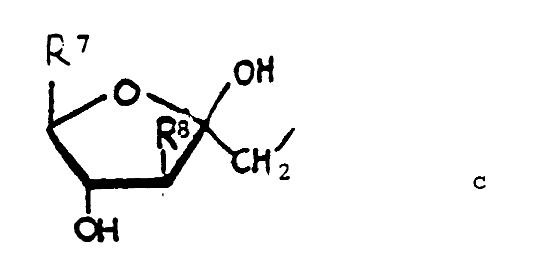 dans laquelle R₇ est un groupe carboxyle, ester, acyle, amide, nitrile, trihalogénométhyle, ou un groupe hétérocyclique, et R₈ est un groupe hydroxyle, un groupe amine, ou un groupe thiol,
ou bien
- une structure de formule (d) ci-après, dans laquelle R₉, R₁₀, R₁₁, représentent des groupes alkyle, acyle ou aryle, et R₁₂ représente un groupe hydroxyle, amine ou alcoxyle, 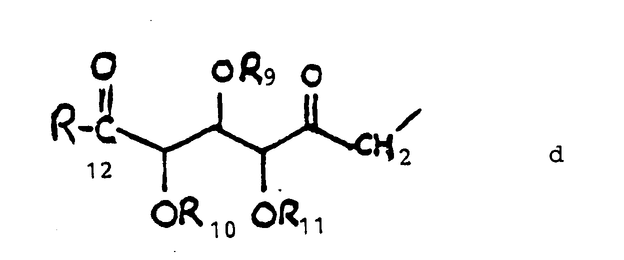
et en ce qu'il comprend une étape au cours de laquelle on soumet le produit de condensation d'un macrolide polyénique et d'un sucre réducteur, à l'action d'un catalyseur pris dans le groupe constitué par les acides de Brönsted et les catalyseurs à méthylène actif, dans des conditions permettant l'induction d'un réarrangement d'Amadori.

2. Procédé selon la revendication 1, caractérisé en ce que le dérivé préparé est sous forme de sel d'un acide de Brönsted.

3. Procédé selon la revendication 2, caractérisé en ce que le dérivé préparé est sous forme de sel mixte d'oxalate et d'ammonium.

4. Procédé selon la revendication 1, caractérisé en ce que le dérivé préparé est sous forme de sel de N-méthyl glucamine.

5. Procédé selon l'une quelconque des revendications 1 à 4, caractérisé en ce que le macrolide polyénique est l'Amphotéricine B.

6. Procédé selon l'une quelconque des revendications 1 à 5, caractérisé en ce que le dérivé préparé est le 1-amino-1-désoxy-D-arabino-glucuronyl-AmB répondant à la formule (IX) suivante : 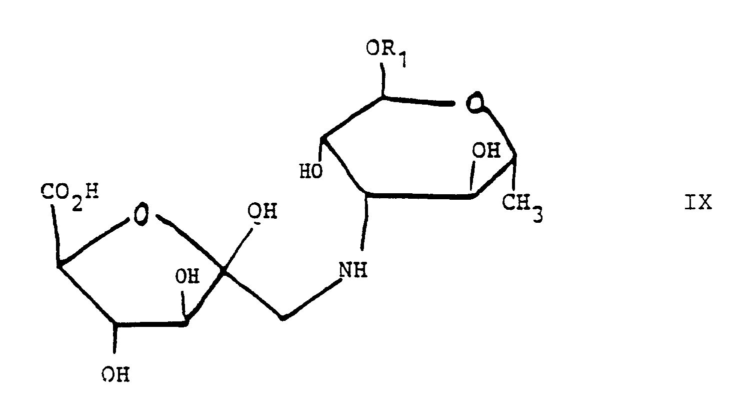 ou son sel de N-méthyl glucamine, ou son sel mixte d'oxalate et d'ammonium.

7. Procédé selon l'une quelconque des revendications 1 à 5, caractérisé en ce que le dérivé préparé est le 1-amino-1-désoxy-D-arabino-glucoronamidyl-AMB répondant à la formule (X) ci-après : 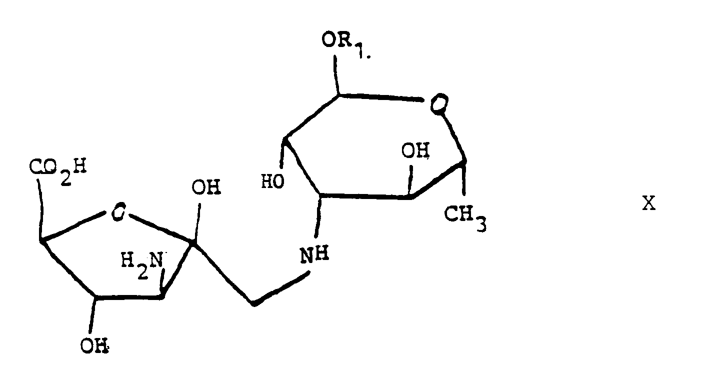 ou son sel de N-méthyl-glucamine, ou son sel mixte d'oxalate et d'ammonium.

8. Procédé selon l'une quelconque des revendications 1 à 5, caractérisé en ce que le dérivé préparé est le 1-désoxy-1-amino-4,6-0-benzylidène-D-fructosyl AMB de formule (VI) ci-après : 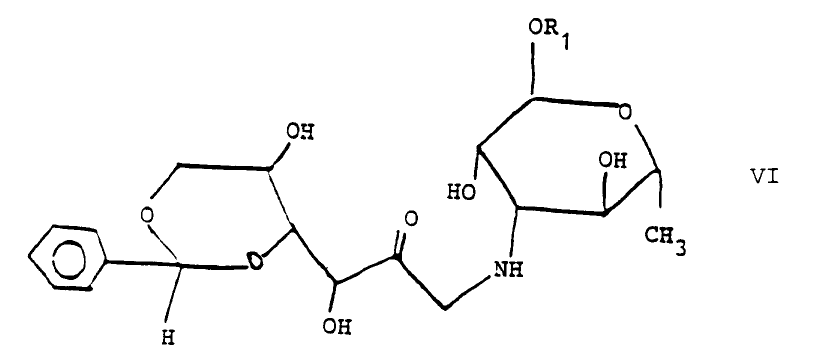 ou son sel de N-méthyl-glucamine, ou son sel mixte d'oxalate et d'ammonium.

9. Procédé de préparation selon l'une quelconque des revendications 1 à 8, caractérisé en ce qu'il comprend la combinaison des étapes suivantes, dans l'ordre suivant :
a) une étape de condensation d'un macrolide polyénique et d'un sucre réducteur, éventuellement substitué, apte à effectuer un réarrangement d'Amadori;
b) une étape d'induction du réarrangement d'Amadori par un catalyseur pris dans le groupe constitué par les acides de Brönsted et les catalyseurs à méthylène actif;
c) une étape de séparation du dérivé réarrangé du milieu réactionnel.

10. Procédé selon la revendication 9, caractérisé en ce qu'il comprend en outre une étape de préparation du sel de N-méthyl-glucamine du dérivé réarrangé.

11. Procédé selon la revendication 9, caractérisé en ce que le sucre réducteur est le D-glucopyranose ou un de ses dérivés.

12. Procédé selon la revendication 9, caractérisé en ce que le sucre réducteur est l'acide β-D-glucuronique ou un de ses dérivés.

13. Procédé de préparation d'un médicament, caractérisé en ce que l'on incorpore audit médicament, en tant que principe actif, un dérivé de macrolide polyénique basique résultant du procédé défini dans l'une quelconque des revendications 1 à 8.

## Claims (Claims for the following Contracting State(s): DE, GB, FR, IT, NL, SE, LI, CH, BE, AT, LU, DK)

1. Basic polyene macrolide derivatives, characterized in that they correspond to the general formula (I) below : 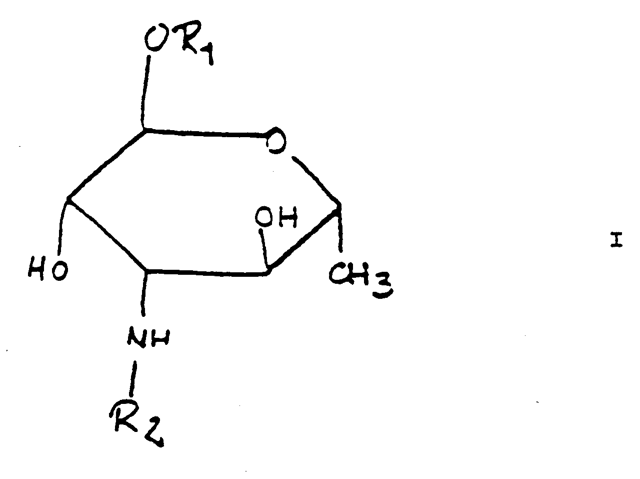 in which :
R₁ denotes the macrocyclic part of a polyene macrolide and
R₂ represents :
- a structure corresponding to the formula (a) below : 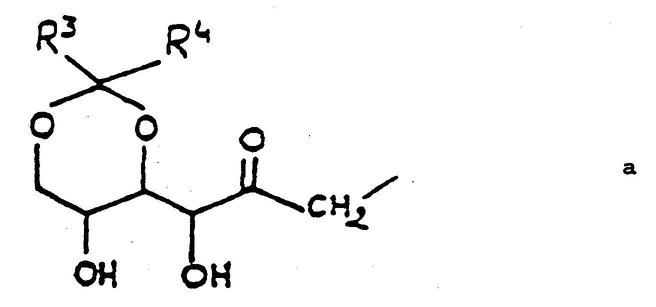 in which R₃ represents a hydrogen atom or a methyl group, and R₄ represents a hydrogen atom, a methyl, trichloromethyl, tert-butyl, propyl, benzyl or phenyl group, or a phenyl group substituted by one or more methoxy, nitro or phosphate groups, or, alternatively, R₃ and R₄ are the linking units of a cycloalkyl group, or, alternatively,
- a structure corresponding to the formula (b) below : 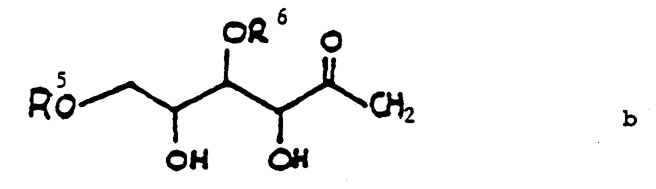 in which R₅ and R₆ are alkyl, aryl, or acyl groups, or, alternatively,
- a structure corresponding to the formula (c) below : 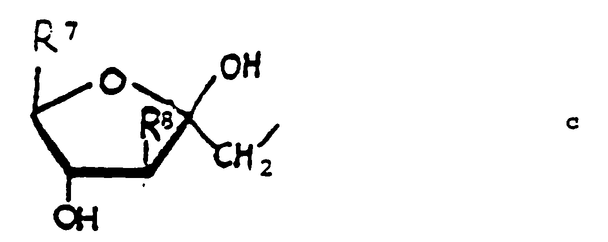 in which R₇ is a carboxyl, ester, acyl, amide, nitrile or trihalomethyl group, or is a heterocyclic group, and R₈ is a hydroxyl group, an amino group, or a mercapto group, or, alternatively,
- a structure of formula (d) below, in which R₉, R₁₀, R₁₁ represent alkyl, acyl or aryl groups, and R₁₂ represents a hydroxyl, amine or alkoxy group, 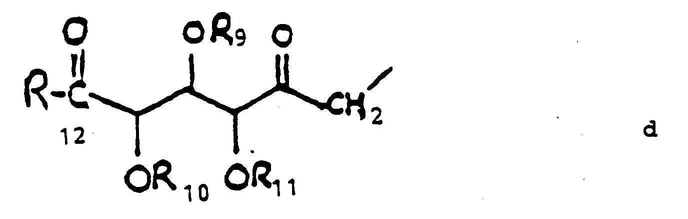
and in that it is capable of being obtained by a process comprising a step during which the condensation product of a polyene macrolide with a reducing sugar is subjected to the action of a catalyst taken from the group comprising Brönsted acids and active methylene catalysts, under conditions making possible the induction of an Amadori rearrangement.

2. Derivative according to Claim 1, characterized in that said derivative is in the form of a salt of a Brönsted acid.

3. Derivative according to Claim 2, characterized in that said derivative is in the form of a mixed oxalate and ammonium salt.

4. Derivative according to Claim 1, characterized in that said derivative is in the form of an N-methylglucamine salt.

5. Derivative according to any one of Claims 1 to 4, characterized in that the polyene macrolide is amphotericin B.

6. Derivative according to any one of Claims 1 to 5, characterized in that it is 1-amino-1-deoxy-D-arabinoglucuronyl-AmB having the following formula (IX) : 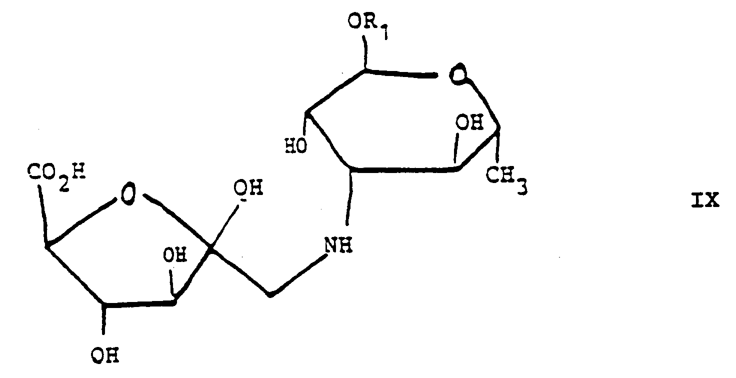 or its N-methylglucamine salt, or its mixed oxalate and ammonium salt.

7. Derivative according to any one of Claims 1 to 5, characterized in that it is 1-amino-1-deoxy-D-arabinoglucuronamidyl-AmB having the formula (X) below : 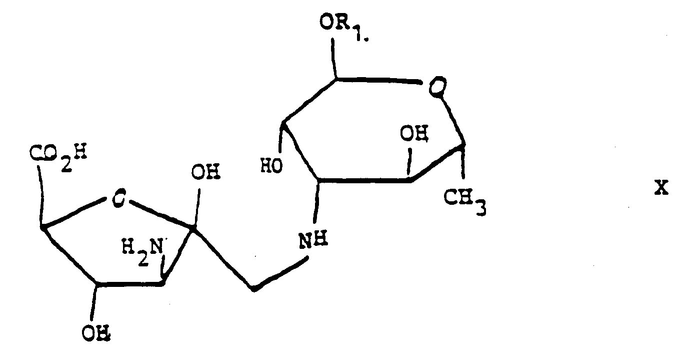 or its N-methylglucamine salt, or its mixed oxalate and ammonium salt.

8. Derivative according to any one of Claims 1 to 5, characterized in that it is 1-deoxy-1-amino-4,6-O-benzylidene-D-fructosyl-AmB of formula (VI) below : 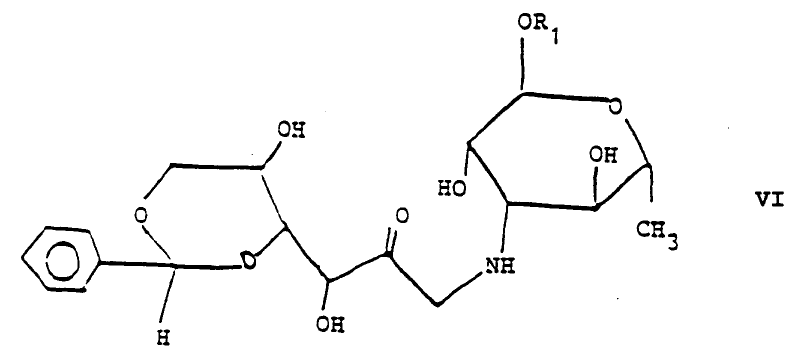 or its N-methylglucamine salt, or its mixed oxalate and ammonium salt.

9. Process for the preparation of a derivative according to any one of Claims 1 to 8, characterized in that it comprises the combination of the following steps in the following order :
a) a condensation step of a polyene macrolide with a reducing sugar, which is substituted or unsubstituted and capable of undergoing an Amadori rearrangement so as to result in the residue R₂ defined in Claim 1;
b) a step inducing the Amadori rearrangement by means of a catalyst taken from the group comprising Brönsted acids and active methylene catalysts,
c) a step of separating the rearranged derivative from the reaction medium.

10. Process according to Claim 9, characterized in that it additionally comprises a step of preparing the N-methylglucamine salt of the rearranged derivative.

11. Process according to Claim 9, characterized in that the reducing sugar is D-glucopyranose or one of its derivatives.

12. Process according to Claim 9, characterized in that the reducing sugar is β-D-glucuronic acid or one of its derivatives.

13. Drugs characterized in that they comprise as active principle a derivative according to any one of Claims 1 to 8.

## Claims (Claims for the following Contracting State(s): ES, GR)

1. Process for the preparation of basic polyene macrolide derivatives, characterized in that there are prepared derivatives corresponding to the general formula (I) below : in which : 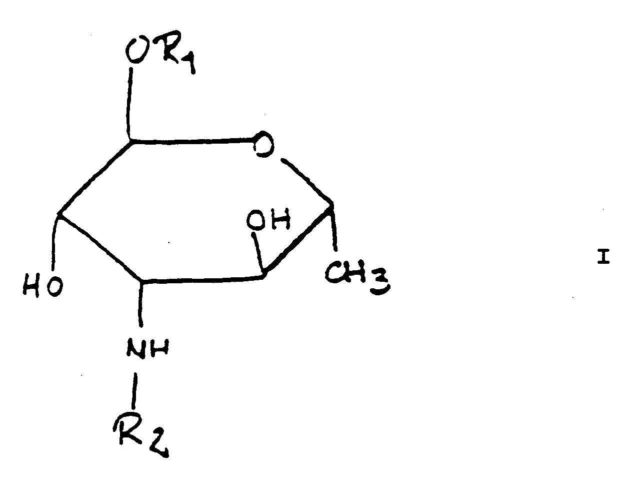
R₁ denotes the macrocyclic part of a polyene macrolide and
R₂ represents :
- a structure corresponding to the formula (a) below : 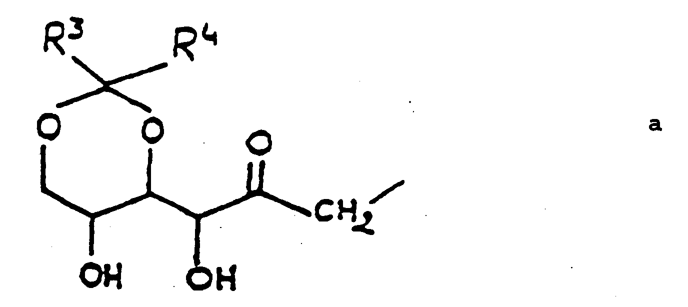 in which R₃ represents a hydrogen atom or a methyl group, and R₄ represents a hydrogen atom, a methyl, trichloromethyl, tert-butyl, propyl, benzyl or phenyl group, or a phenyl group substituted by one or more methoxy, nitro or phosphate groups, or, alternatively, R₃ and R₄ are the linking units of a cycloalkyl group,
or, alternatively,
- a structure corresponding to the formula (b) below : 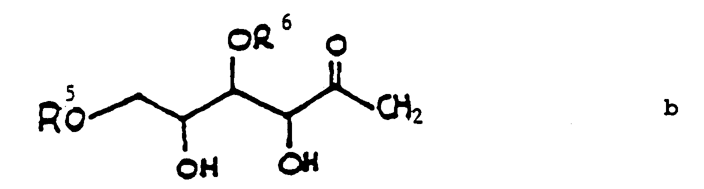 in which R₅ and R₆ are alkyl, aryl, or acyl groups, or, alternatively,
- a structure corresponding to the formula (c) below : 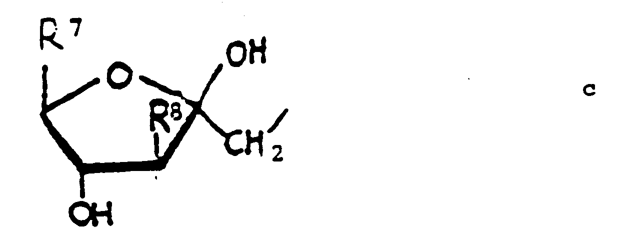 in which R₇ is a carboxyl, ester, acyl, amide, nitrile or trihalomethyl group, or is a heterocyclic group, and R₈ is a hydroxyl group, an amino group, or a mercapto group, or, alternatively,
- a structure of formula (d) below, in which R₉, R₁₀, R₁₁ represent alkyl, acyl or aryl groups, and R₁₂ represents a hydroxyl, amine or alkoxy group, 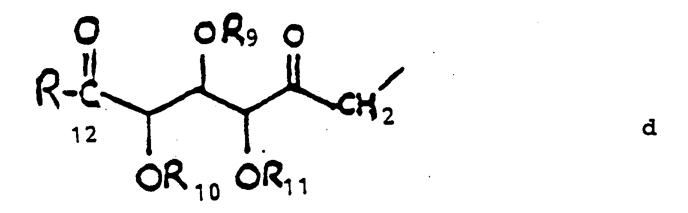
and in that it comprises a step during which the condensation product of a polyene macrolide with a reducing sugar is subjected to the action of a catalyst taken from the group comprising Brönsted acids and active methylene catalysts, under conditions making possible the induction of an Amadori rearrangement.

2. Process according to Claim 1, characterized in that the derivative prepared is in the form of a salt of a Brönsted acid.

3. Process according to Claim 2, characterized in that the derivative prepared is in the form of a mixed oxalate and ammonium salt.

4. Process according to Claim 1, characterized in that the derivative prepared is in the form of an N-methylglucamine salt.

5. Process according to any one of Claims 1 to 4, characterized in that the polyene macrolide is amphotericin B.

6. Process according to any one of Claims 1 to 5, characterized in that the derivative prepared is 1-amino-1-deoxy-D-arabinoglucuronyl-AmB having the following formula (IX) : 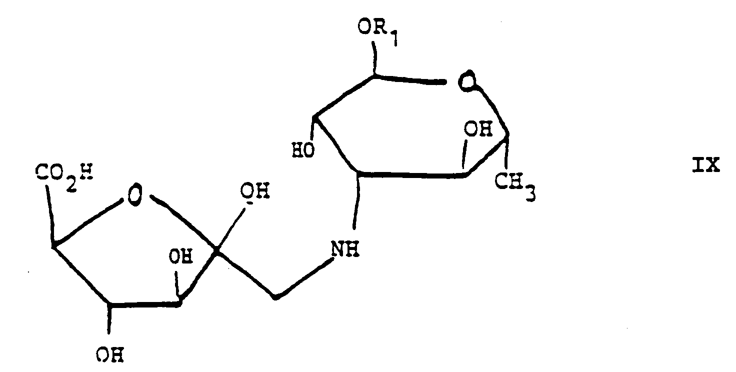 or its N-methylglucamine salt, or its mixed oxalate and ammonium salt.

7. Process according to any one of Claims 1 to 5, characterized in that the derivative prepared is 1-amino-1-deoxy-D-arabinoglucuronamidyl-AmB having the formula (X) below: 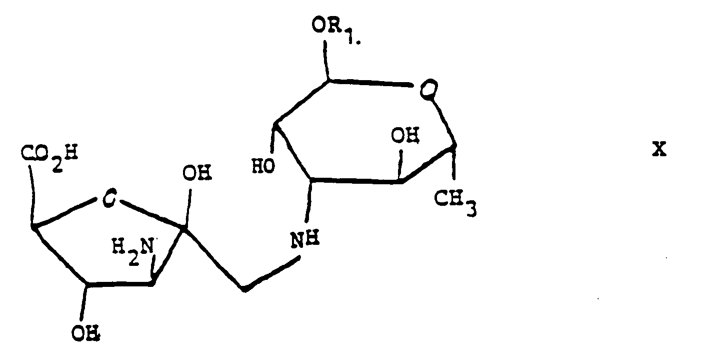 or its N-methylglucamine salt, or its mixed oxalate and ammonium salt.

8. Process according to any one of Claims 1 to 5, characterized in that the derivative prepared is 1-deoxy-1-amino-4,6-O-benzylidene-D-fructosyl-AmB of formula (VI) below : 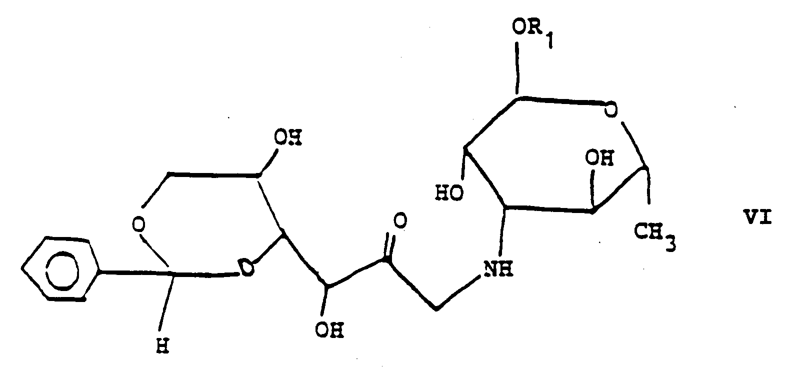 or its N-methylglucamine salt, or its mixed oxalate and ammonium salt.

9. Preparation process according to any one of Claims 1 to 8, characterized in that it comprises the combination of the following steps in the following order:
a) a condensation step of a polyene macrolide with a reducing sugar, which is substituted or unsubstituted and capable of undergoing an Amadori rearrangement;
b) a step inducing the Amadori rearrangement by means of a catalyst taken from the group comprising Brönsted acids and active methylene catalysts,
c) a step of separating the rearranged derivative from the reaction medium.

10. Process according to Claim 9, characterized in that it additionally comprises a step of preparing the N-methylglucamine salt of the rearranged derivative.

11. Process according to Claim 9, characterized in that the reducing sugar is D-glucopyranose or one of its derivatives.

12. Process according to Claim 9, characterized in that the reducing sugar is β-D-glucuronic acid or one of its derivatives.

13. Process for the preparation of a drug, characterized in that there is incorporated in the said drug, as active principle, a basic polyene macrolide derivative resulting from the process defined in any one of Claims 1 to 8.

## Patentansprüche (Patentansprüche für folgende(n) Vertragsstaat(en): DE, GB, FR, IT, NL, SE, LI, CH, BE, AT, LU, DK)

1. Derivate von basischen Polyenmakroliden, dadurch **gekennzeichnet**, daß sie der nachstehenden allgemeinen Formel (I) entsprechen worin 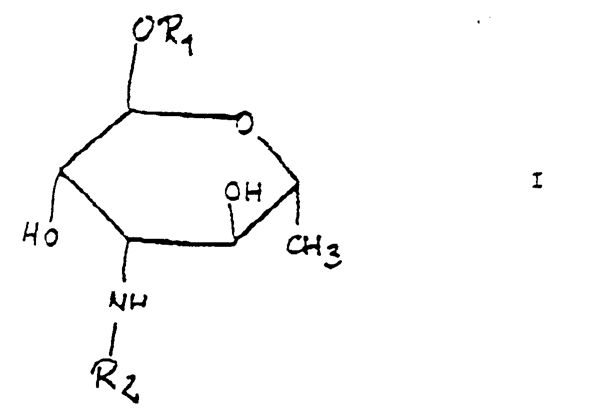
R₁ den makrocyclischen Teil eines Polyenmakrolids bezeichnet, und
R₂
- eine Struktur der nachstehenden Formel (a) bedeutet, 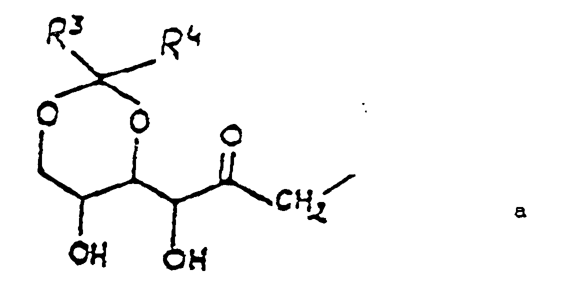 worin R₃ ein Wasserstoffatom oder eine Methylgruppe bedeutet, und R₄ ein Wasserstoffatom, eine Methyl-Trichlormethyl-, tert.-Butyl-, Propyl-, Benzyl-, Phenyl- oder eine durch eine oder mehrere Methoxy-, Nitro- oder Phosphatgruppe(n) substituierte Phenylgruppe bedeutet, oder R₃ und R₄ Glieder eines Cycloalkylrests sind, oder
- eine Struktur der nachstehenden Formel (b) 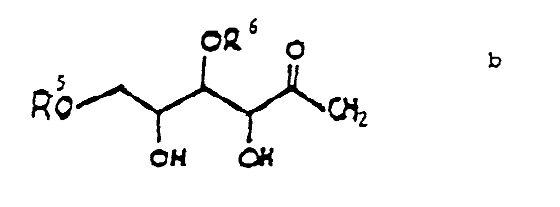 worin R₅ und R₆ Alkyl-, Aryl- oder Acylreste sind, oder
- eine Struktur der nachstehenden Formel (c) 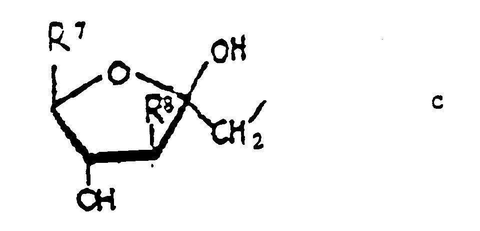 worin R₇ eine Carboxyl-, Ester-, Acyl-, Amid-, Nitril-, Trihalogenmethylgruppe oder ein heterocyclischer Rest ist, und R₈ eine Hydroxylgruppe, eine Amingruppe oder eine Thiolgruppe ist, oder
- eine Struktur der nachstehenden Formel (d), worin R₉, R₁₀, R₁₁ Alkyl-, Acyl- oder Arylreste bedeuten, und R₁₂ eine Hydroxyl-, Amin-, oder Alkoxylgruppe bedeutet, 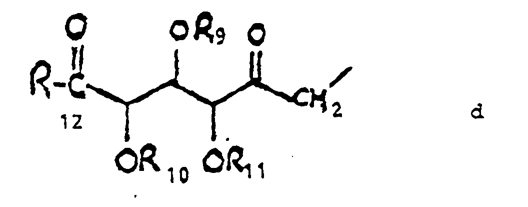 bedeutet,
und daß sie durch ein Verfahren erhalten werden können, das eine Stufe umfaßt, im Verlauf derer man das Kondensationsprodukt eines Polyenmakrolids und eines reduzierenden Zuckers mit einem Katalysator aus der Gruppe Brönsted-Säuren und Katalysatoren mit aktivem Methylen unter Bedingungen umsetzt, die die Induktion einer Amadori-Umlagerung erlauben.

2. Derivat nach Anspruch 1, dadurch **gekennzeichnet**, daß das Derivat in Form eines Salzes einer Brönsted-Säure vorliegt.

3. Derivat nach Anspruch 2, dadurch **gekennzeichnet**, daß das Derivat in Form eines gemischten Ammoniumoxalatsalzes vorliegt.

4. Derivat nach Anspruch 1, dadurch **gekennzeichnet**, daß das Derivat in Form eines N-Methylglucaminsalzes vorliegt.

5. Derivat nach einem der Ansprüche 1 bis 4, dadurch **gekennzeichnet**, daß das Polyenmakrolid Amphotericin B ist.

6. Derivat nach einem der Ansprüche 1 bis 5, dadurch **gekennzeichnet**, daß es 1-Amino-1-desoxy-D-arabinoglucuronyl-AmB der nachstehenden Formel (IX) 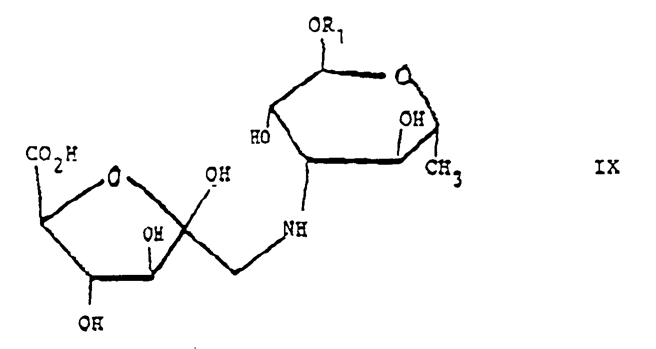 oder dessen N-Methylglucaminsalz oder dessen gemischtes Ammoniumoxalatsalz ist.

7. Derivat nach einem der Ansprüche 1 bis 5, dadurch **gekennzeichnet**, daß es 1-Amino-1-desoxy-D-arabinoglucuronamidyl-AmB der nachstehenden Formel (X) 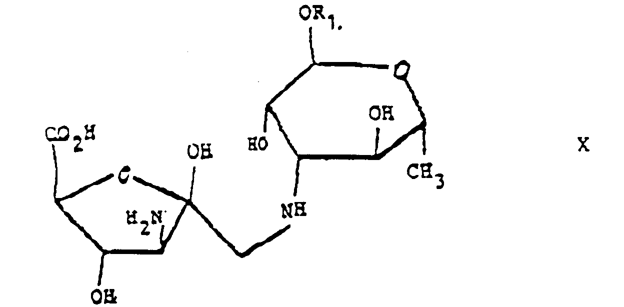 oder dessen N-Methylglucaminsalz oder dessen gemischtes Ammoniumoxalatsalz ist.

8. Derivat nach einem der Ansprüche 1 bis 5, dadurch **gekennzeichnet**, daß es 1-Desoxy-1-amino-4,6-O-benzyliden-D-fructosyl-AmB der nachstehenden Formel(VI) 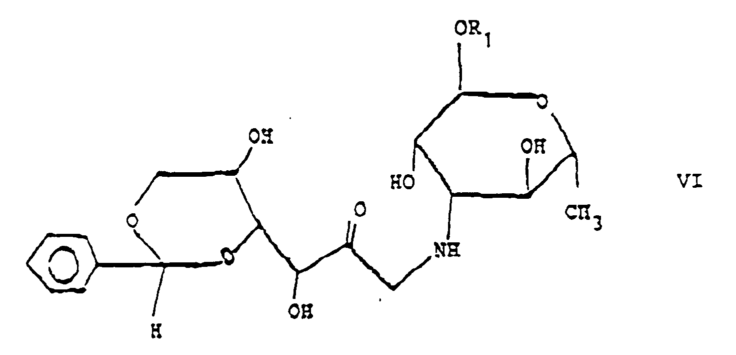 oder dessen N-Methylglucaminsalz oder dessen gemischtes Ammoniumoxalatsalz ist.

9. Verfahren zur Herstellung eines Derivats nach einem der Ansprüche 1 bis 8, dadurch **gekennzeichnet**, daß es die Kombination der folgenden Stufen in der folgenden Reihenfolge umfaßt:
a) eine Stufe der Kondensation eines Polyenmakrolids und eines reduzierenden, gegebenenfalls substituierten, Zuckers mit der Eignung, eine Amadori-Umlagerung zu bewirken, so daß sie am vorstehend in Anspruch 1 definierten Rest R₂ stattfindet,
b) eine Stufe der Induktion einer Amadori-Umlagerung durch einen Katalysator aus der Gruppe Brönsted-Säuren und Katalysatoren mit aktivem Methylen,
c) eine Stufe der Abtrennung des umgelagerten Derivats aus dem Reaktionsmedium.

10. Verfahren nach Anspruch 9, dadurch **gekennzeichnet**, daß es weiterhin eine Stufe der Herstellung des N-Methylglucaminsalzes des umgelagerten Derivats umfaßt.

11. Verfahren nach Anspruch 9, dadurch **gekennzeichnet**, daß der reduzierende Zucker D-Glucopyranose oder eines ihrer Derivate ist.

12. Verfahren nach Anspruch 9, dadurch **gekennzeichnet**, daß der reduzierende Zucker β-D-Glucuronsäure oder eines ihrer Derivate ist.

13. Arzneimittel, dadurch **gekennzeichnet**, daß sie als Wirkstoff ein Derivat nach einem der Ansprüche 1 bis 8 enthalten.

## Patentansprüche (Patentansprüche für folgende(n) Vertragsstaat(en): ES, GR)

1. Verfahren zur Herstellung von Derivaten von basischen Polyenmakroliden, dadurch **gekennzeichnet**,
daß man Derivate der nachstehenden allgemeinen Formel (I) herstellt, worin 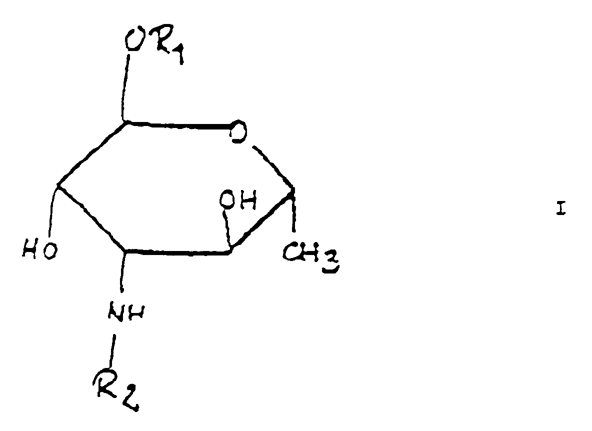
R₁ den makrocyclischen Teil eines Polyenmakrolids bezeichnet, und
R₂
- eine Struktur der nachstehenden Formel (a) bedeutet, 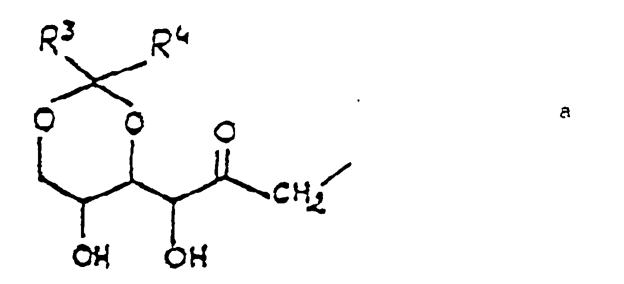 worin R₃ ein Wasserstoffatom oder eine Methylgruppe bedeutet, und R₄ ein Wasserstoffatom, eine Methyl-Trichlormethyl-, tert.-Butyl-, Propyl-, Benzyl-, Phenyl- oder eine durch eine oder mehrere Methoxy-, Nitro- oder Phosphatgruppe(n) substituierte Phenylgruppe bedeutet, oder R₃ und R₄ Glieder eines Cycloalkylrests sind, oder
- eine Struktur der nachstehenden Formel (b) 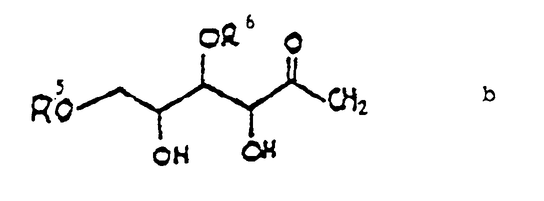 worin R₅ und R₆ Alkyl-, Aryl- oder Acylreste sind, oder
- eine Struktur der nachstehenden Formel (c) 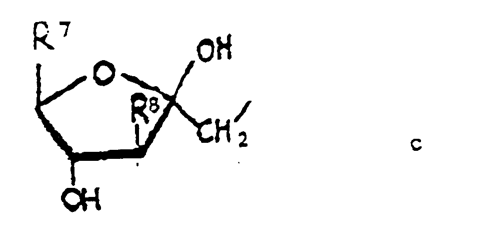 worin R₇ eine Carboxyl-, Ester-, Acyl-, Amid-, Nitril-, Trihalogenmethylgruppe oder ein heterocyclischer Rest ist, und R₈ eine Hydroxylgruppe, eine Amingruppe oder eine Thiolgruppe ist, oder
- eine Struktur der nachstehenden Formel (d), worin R₉, R₁₀, R₁₁ Alkyl-, Acyl- oder Arylreste bedeuten, und R₁₂ eine Hydroxyl-, Amin-, oder Alkoxylgruppe bedeutet, 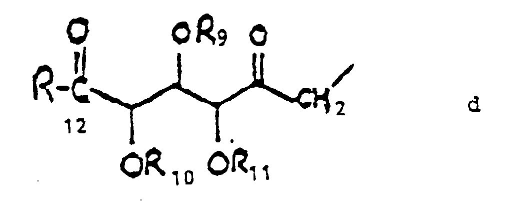 bedeutet,
und daß es eine Stufe umfaßt, im Verlauf derer man das Kondensationsprodukt eines Polyenmakrolids und eines reduzierenden Zuckers mit einem Katalysator aus der Gruppe Brönsted-Säuren und Katalysatoren mit aktivem Methylen unter Bedingungen umsetzt, die die Induktion einer Amadori-Umlagerung erlauben.

2. Verfahren nach Anspruch 1, dadurch **gekennzeichnet**, daß das hergestellte Derivat in Form eines Salzes einer Brönsted-Säure vorliegt.

3. Verfahren nach Anspruch 2, dadurch **gekennzeichnet**, daß das hergestellte Derivat in Form eines gemischten Ammoniumoxalatsalzes vorliegt.

4. Verfahren nach Anspruch 1, dadurch **gekennzeichnet**, daß das hergestellte Derivat in Form eines N-Methylglucaminsalzes vorliegt.

5. Verfahren nach einem der Ansprüche 1 bis 4, dadurch **gekennzeichnet**, daß das Polyenmakrolid Amphotericin B ist.

6. Verfahren nach einem der Ansprüche 1 bis 5, dadurch **gekennzeichnet**, daß das hergestellte Derivat 1-Amino-1-desoxy-D-arabinoglucuronyl-AmB der nachstehenden Formel (IX) 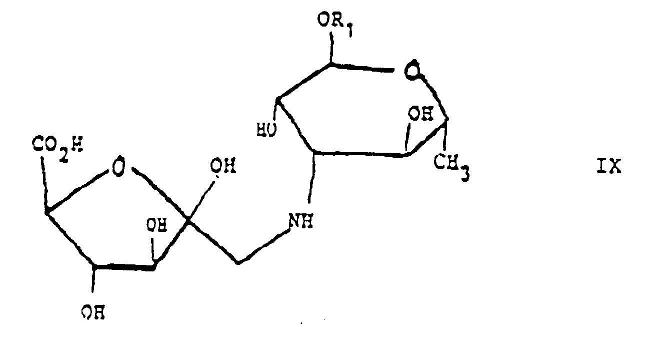 oder dessen N-Methylglucaminsalz oder dessen gemischtes Ammoniumoxalatsalz ist.

7. Verfahren nach einem der Ansprüche 1 bis 5, dadurch **gekennzeichnet**, daß das hergestellte Derivat 1-Amino-1-desoxy-D-arabinoglucuronamidyl-AmB der nachstehenden Formel (X) 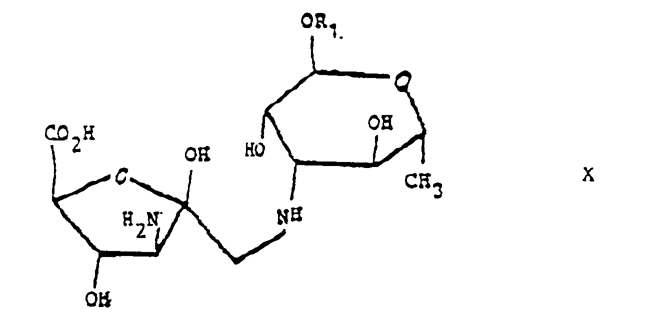 oder dessen N-Methylglucaminsalz oder dessen gemischtes Ammoniumoxalatsalz ist.

8. Verfahren nach einem der Ansprüche 1 bis 5, dadurch **gekennzeichnet**, daß das hergestellte Derivat 1-Desoxy-1-amino-4,6-O-benzyliden-D-fructosyl-AmB der nachstehenden Formel(VI) 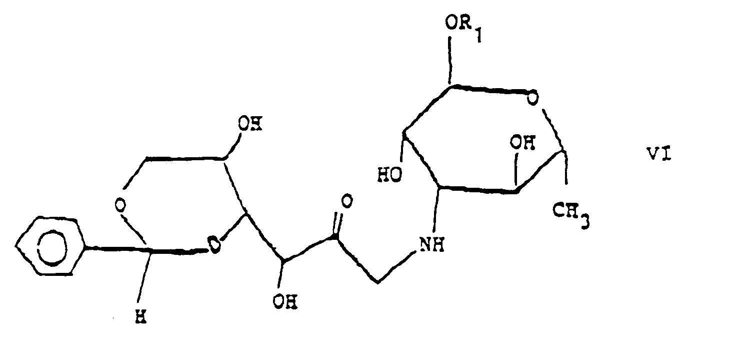 oder dessen N-Methylglucaminsalz oder dessen gemischtes Ammoniumoxalatsalz ist.

9. Verfahren zur Herstellung nach einem der Ansprüche 1 bis 8, dadurch **gekennzeichnet**, daß es die Kombination der folgenden Stufen in der folgenden Reihenfolge umfaßt:
a) eine Stufe der Kondensation eines Polyenmakrolids und eines reduzierenden, gegebenenfalls substituierten, Zuckers mit der Eignung, eine Amadori-Umlagerung zu bewirken,
b) eine Stufe der Induktion einer Amadori-Umlagerung durch einen Katalysator aus der Gruppe Brönsted-Säuren und Katalysatoren mit aktivem Methylen,
c) eine Stufe der Abtrennung der umgelagerten Derivats aus dem Reaktionsmedium.

10. Verfahren nach Anspruch 9, dadurch **gekennzeichnet**, daß es weiterhin eine Stufe der Herstellung des N-Methylglucaminsalzes des umgelagerten Derivats umfaßt.

11. Verfahren nach Anspruch 9, dadurch **gekennzeichnet**, daß der reduzierende Zucker D-Glucopyranose oder eines ihrer Derivate ist.

12. Verfahren nach Anspruch 9, dadurch **gekennzeichnet**, daß der reduzierende Zucker β-D-Glucuronsäure oder eines ihrer Derivate ist.

13. Verfahren zur Herstellung eines Arzneimittels, dadurch **gekennzeichnet**, daß man in das Arzneimittel als Wirkstoff ein Derivat eines basischen Polyenmakrolids, das aus dem Verfahren nach einem der Ansprüche 1 bis 8 hervorgeht, einarbeitet.
